# EUROPÄISCHE PATENTSCHRIFT

(11) **EP 0 944 569 B1**
(45) Veröffentlichungstag und Bekanntmachung des Hinweises auf die Patenterteilung: **10.04.2002**
(21) Anmeldenummer: 97953752.9
(22) Anmeldetag: 08.12.1997
(51) Int. Cl.: C07C 43/225, C07C 43/23, C09K 11/07

(54) **POLYMERISIERBARE BIARYLE, VERFAHREN ZUR HERSTELLUNG UND DEREN VERWENDUNG**
POLYMERIZABLE BIARYLS, METHOD FOR THE PRODUCTION AND USE THEREOF
BIARYLES POLYMERISABLES, LEUR PROCEDE DE PREPARATION ET LEUR UTILISATION

(30) Priorität: 11.12.1996 DE 19651439
(43) Veröffentlichungstag der Anmeldung: 29.09.1999
(73) Patentinhaber: Celanese Ventures GmbH, 65926 Frankfurt am Main (DE)
(72) Erfinder: SPREITZER, Hubert, D-65929 Frankfurt am Main (DE); KREUDER, Willi, D-55126 Mainz (DE); BECKER, Heinrich, D-61479 Glashütten (DE); KRAUSE, Jochen, D-60594 Frankfurt am Main (DE)
(86) Internationale Anmeldenummer: EP9706830
(87) Internationale Veröffentlichungsnummer: WO9825874

(56) Entgegenhaltungen:
- WO-A-90/13148

## Beschreibung

Derivate des Poly(p-phenylenvinylen) sind seit längerem als Elektrolumineszenz (EL)-Materialien bekannt (siehe z.B. WO-A 90/13148). Ist in diesen Polymeren die Phenylengruppe durch einen oder mehrere weitere Arylreste substituiert, ergeben sich EL-Materialien mit einem ganz besonderen Eigenschaftsspektrum, die sich insbesondere zur Erzeugung grüner Elektrolumineszenz eignen.
Ausgangsverbindungen für solche Polymere sind Biarylmonomere, die an einem Ring zwei zur Polymerisation befähigende Gruppen, wie CH₂Br, in 1,4-Stellung enthalten.

Um Polymere mit praxistauglichen Eigenschaften für die Anwendung in EL-Displays herstellen zu können, benötigt man die entsprechenden Monomere in außergewöhnlich hoher Reinheit. Es ist zudem eine Anforderung der industriellen Praxis, daß eine entsprechende Reinheit in möglichst wenigen, einfachen und kostengünstigen Schritten zu erzielen ist.

Da zudem die Entwicklung von Elektrolumineszenzmaterialien, insbesondere auf Grundlage von Polymeren, noch in keiner Weise als abgeschlossen betrachtet werden kann, sind die Hersteller von Beleuchtungs- und Anzeigevorrichtungen nach wie vor an den unterschiedlichsten Elektrolumineszenzmaterialien für solche Vorrichtungen interessiert.

Daraus ergibt sich insbesondere für die industrielle Praxis die Anforderung, daß sich nach einem Syntheseverfahren eine breite Palette von Monomeren herstellen lassen sollte.

Aus dem Stand der Technik ist es bekannt, zur Polymerisation befähigende Gruppen durch elektrophile Substitution in ein Biaryl einzuführen (vgl. z.B. G. Subramaniam et al., Synthesis, 1992, 1232 und v. Braun, Chem. Ber. 1937, 70, 979).

Diese Route ist aber nicht allgemein anwendbar, da die Substitution in der Regel in beiden Arylsystemen erfolgt, was zumindest eine aufwendige Auftrennung der verschiedenen Produkte erfordert.

Die Bromierung von 4,4"-Dihexyloxy-2',5'-dimethyl-p-terphenyl mit N-Bromsuccinimid ist von J. Andersch et al., J. Chem. Soc. Commun. 1995, 107, beschrieben. Hierbei kommt es jedoch neben der Bromierung der Methylgruppen auch zu einer der Alkoxyketten (siehe Vergleichsversuch V2 und K.L. Platt und F. Setiabudi, J. Chem. Soc. Perkin Trans. 1, 1992, 2005).

W.E. Bachmann und N.C. Denno, J. Am. Chem. Soc. 1949, 71, 3062 beschreiben die Synthese von Biarylderivaten durch 4+2 Cycloaddition eines Styrols mit einem Dien-1,6-dicarbonsäurederivat und anschließende Dehydrierung des gebildeten Sechsringes zum Aromaten.
Nachteilig, neben Preis und Zugänglichkeit der Ausgangsverbindungen, ist hierbei die Tatsache, daß die Bedingungen der Dehydrierungsreaktion nicht von allen funktionellen Gruppen toleriert werden und daher das Substitutionsmuster erheblich eingeschränkt wird. Daher bestand weiterhin Bedarf an einem allgemeinen Syntheseverfahren, das die obengenannten Anforderungen erfüllt.
Es wurde nun in funktionalisierten Aryl-1,4-bismethanolen und -biscarbonsäureestern breit- und einfach zugänglich Ausgangsprodukte gefunden, die durch die spezielle Reaktionsfolge, palladiumkatalytierte Kupplung mit einer zweiten Arylkomponente und Umwandlung der Alkohol bzw. Esterfunktionen in zur Polymerisation geeignete Gruppen, leicht in hoher Reinheit in die gewünschten Monomere überführt werden können.

Gegenstand der Erfindung ist daher ein Verfahren zur Herstellung eines polymerisierbaren Biarylderivats der Formel (I), 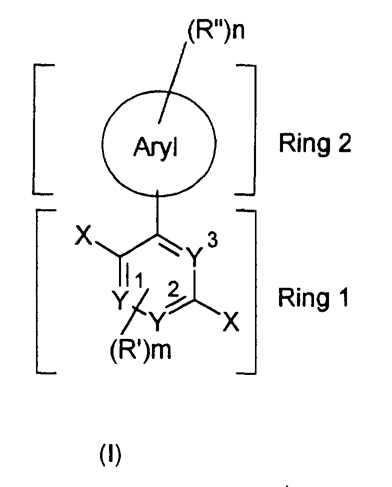 wobei die Symbole und Indizes folgende Bedeutungen haben:
- X :: -CH₂Z, -CHO;
- Y¹, Y², Y³:: gleich oder verschieden, CH, N;
- Z :: gleich oder verschieden, l, Cl, Br, CN, SCN, NCO, PO(OR¹)₂, PO(R²)₂, P(R³)₃⁺A⁻;
- Aryl :: eine Arylgruppe mit 4 bis 14 C-Atomen;
- R', R" :: gleich oder verschieden CN, F, Cl, eine geradkettige oder verzweigte oder cyclische Alkyl- oder Alkoxygruppe mit 1 bis 20 C-Atomen, wobei ein oder mehrere nicht benachbarte CH₂-Gruppen durch -O-, -S-, -CO-, -COO-, -O-CO-, -NR⁴-, -(NR⁵R⁶)⁺-A⁻, oder -CONR⁷- ersetzt sein können und wobei ein oder mehrere H-Atome durch F ersetzt sein können, oder eine Arylgruppe mit 4 bis 14 C-Atome, die durch einen oder mehrere, nicht aromatische Reste R' substituiert sein kann;
- R¹,R²,R³,R⁴,R⁵,R⁶,R⁷: gleich oder verschieden aliphatische oder aromatische Kohlenwasserstoffreste mit 1 bis 20 C-Atomen, R⁴ bis R⁷ können auch H sein;
- A^{⊖} :: ein einfach geladenes Anion oder dessen Äquivalent;
- m :: 0,1 oder 2;
- n :: 1, 2, 3, 4 oder 5;
dadurch gekennzeichnet,
A. daß man zwei Arylderivate der Formeln (II) und (III), 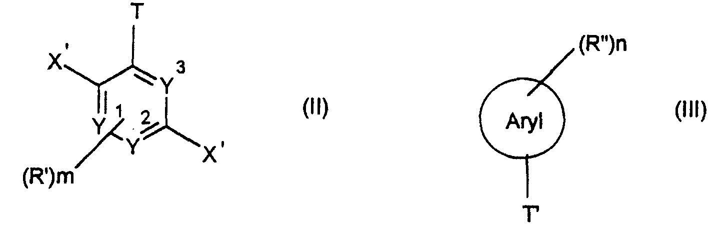 in einem inerten Lösungsmittel in Gegenwart eines Palladiumkatalysators bei einer Temperatur im Bereich von 0°C bis 200°C zu einem Zwischenprodukt der Formel (IV) 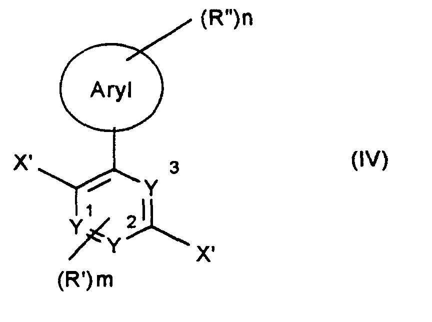 umsetzt,
   wobei die Symbole und Indizes die in Formel (I) angegebenen Bedeutungen haben und
   - X' :: CH₂OH oder COOR⁸;
   eine der Gruppen T, T': Cl, Br, I oder ein Perfluoralkylsulfonatrest, mit vorzugsweise 1 bis 12 C-Atomen,
   und die andere Gruppe T, T': SnR₃, BQ₁Q₂ bedeutet wobei
   - Q₁,Q₂: gleich oder verschieden -OH, C₁-C₄-Alkoxy, C₁-C₄-Alkyl, Phenyl, das gegebenenfalls durch C₁-C₄-Alkyl, C₁-C₄-Alkoxy oder Halogen substituiert sein kann, oder Halogen bedeuten oder Q₁ und Q₂ zusammen bilden eine C₁-C₄-Alkylendioxy-Gruppe, die gegebenenfalls durch eine oder zwei C₁-C₄-Alkylgruppen substituiert sein kann; und
   - R⁸: ist gleich oder verschieden H oder eine geradkettige oder verzweigte Alkylgruppe mit 1 bis 12 Kohlenstoffatomen;
B. falls die Gruppe X' in dem Zwischenprodukt der Formel (IV) COOR⁸ bedeutet (IVa), man mit einem Reduktionsmittel zu einem Zwischenprodukt der Formel (IV) reduziert, bei dem X' CH₂OH bedeutet (IVb), und
C. man das so erhaltene Zwischenprodukt der Formel (IVb) nach einer der folgenden Reaktionen umsetzt:
   a) selektive Oxidation zu einer Verbindung der Formel (1) mit X = CHO oder
   b) Austausch der OH-Gruppe gegen ein Halogen oder Pseudohalogen mittels nucleophiler Substitution zu einer Verbindung der Formel (I) mit Z = Cl, Br, I, CN, SCN, OCN; und
D. man gegebenenfalls Verbindungen der Formel (I) mit Z = Cl, Br, I durch Umsetzung mit den entsprechenden phosphororganischen Verbindungen ein Biarylderivat der Formel (I) mit Z = PO(OR¹)₂, PO(R²)₂,
   P(R³)₃⁺A⁻ überführt.

Ein wesentlicher Vorteil des erfindungsgemäßen Verfahrens ist es, daß auf Stufe der Biarylderivate in der Regel eine einfache Reinigung, insbesondere durch Umkristallisation, durchführbar ist.

Zwar erweisen sich die meisten Verbindungen der Formel (IV) mit X'=COOR und der Formel (I) mit X = CH₂Cl, CH₂Br als hochsiedende Öle, diese können jedoch in der Regel aus der Synthese sauber gewonnen werden. Die erfindungsgemäß ausgewählten Kupplungsreaktionen können routinemäßig so gestaltet werden, daß die entstehenden Produkte (IV) mit Reinheiten größer 90 % anfallen. Die Umsetzung zu Bishalogeniden der Formel (I) führt in der Regel nur zu geringer Nebenprodukt-bildung, so daß diese Substanzen mit ähnlicher Reinheit wie die eingesetzten Bisalkohole (IV) anfallen. Diese wiederum stellen in der Regel kristalline Substanzen dar, die sich durch einfache Umkristallisation problemlos zu Reinheiten größer 99% aufreinigen lassen. Analoges gilt auch für die Bisphosphonate und insbesondere Bisphosphoniumsalze der Formel (I). Bei den Bisaldehyden der Formel (I) wird je nach Substitutionsmuster ein hochviskoses Öl oder eine kristalline Substanz gefunden. Wählt man die Reaktionsbedingungen bei der Herstellung erfindungsgemäß (z.B. Swernoxidation), wird der Bisaldehyd ebenfalls in hoher Reinheit direkt aus der Reaktionsmischung erhalten.

Das erfindungsgemäße Verfahren ist ein Schema 1 graphisch dargestellt.

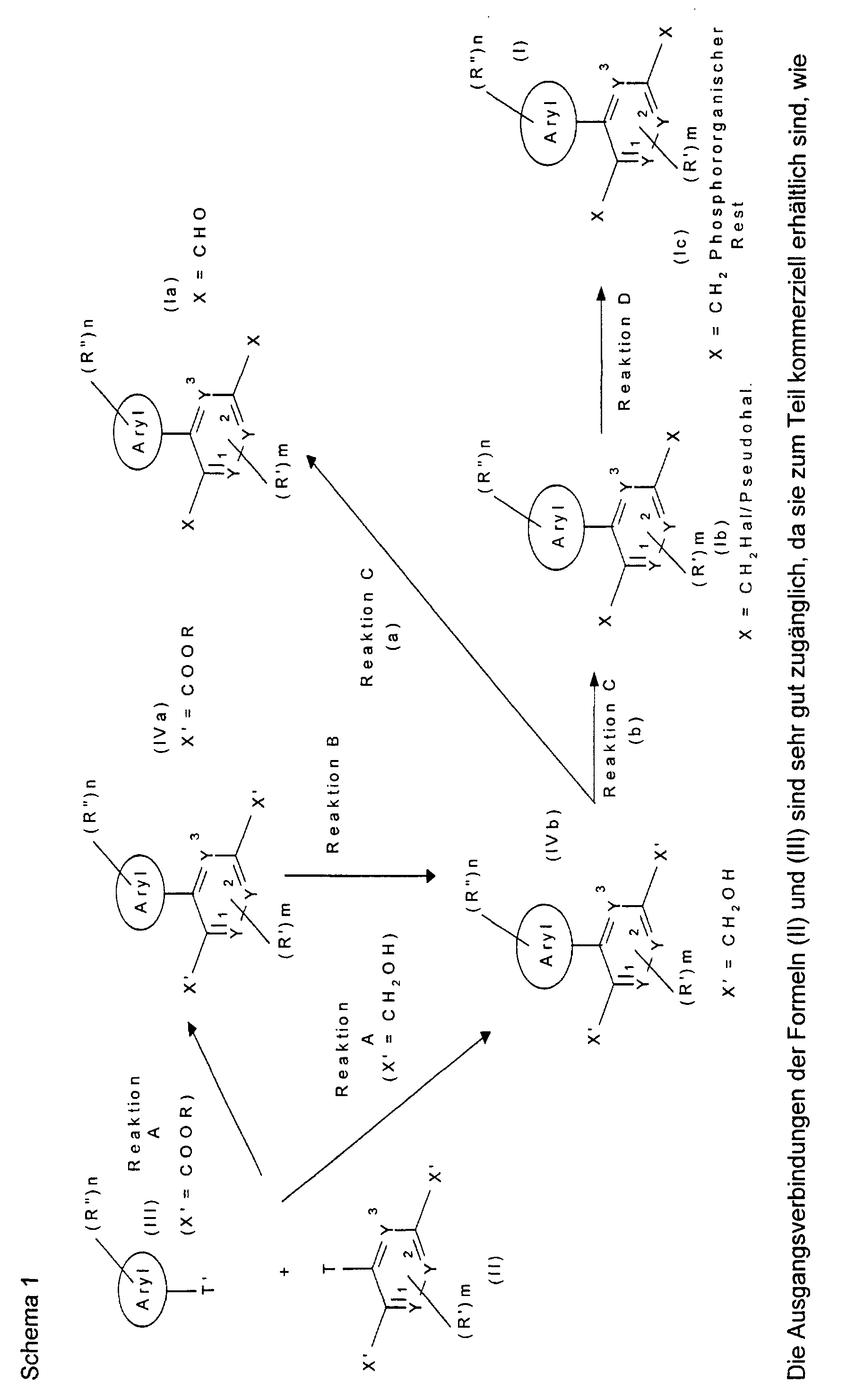

Bromterephthalsäure, oder in einfacher Weise und großer Menge aus kommerziell erhältlichen Verbindungen herstellbar sind.

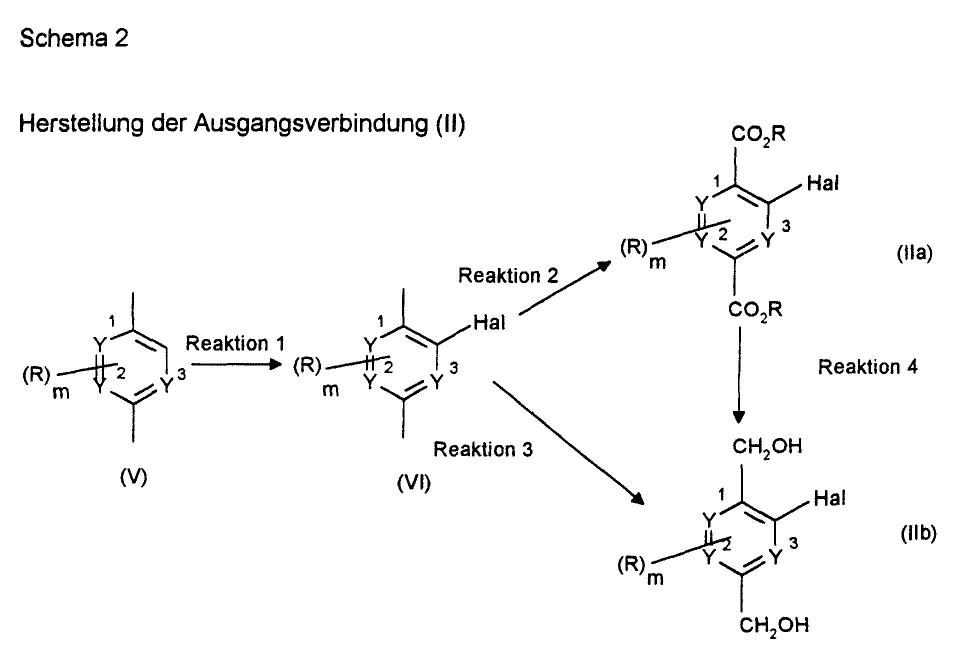

Die Reaktionen in Schema 2 sind wie folgt zu erläutern: 1,4-Dimethyl-Verbindung (V) ist in der Regel kommerziell erhältlich (z. B. p-Xylol, 2,5-Dimethylphenol, 2,5-Dimethylanilin, 2,5-Dimethylbenzonitril, 2,5-Dimethylpyridin) oder aus kommerziell erhältlichen Verbindungen einfach herzustellen (z. B. Alkylierung eines entsprechenden Phenols oder Amins), Verbindung (V) kann nach Standardmethoden am Aromaten halogeniert, z.B. chloriert oder bromiert, werden (siehe z.B. Organikum, VEB Deutscher Verlag der Wissenschaften, 15. Auflage, S. 391 ff., Leipzig 1984). Die damit erhaltenen Verbindungen (VI) sind in guten Ausbeuten und industriellen Mengen zugänglich; zum Teil ist die Verbindung (VI) auch kommerziell erhältlich (z. B. 2-Brom-p-xylol).
(VI) kann, vorzugsweise katalytisch (Kobaltkatalysator, Luftsauerstoff, siehe z.B. EP-A 0 121 684) zu den entsprechenden 1,4-Dicarbonsäuren (IIa) umgesetzt werden. Bei geeigneter Wahl der Reaktionsbedingungen ist dies routinemäßig unabhängig vom Substituenten möglich. Die erhaltenen Säuren, (lla) mit R = H, sind gegebenenfalls nach Standardmethoden in entsprechende Ester (R≠H) überführbar.

Die auf diese Weise nahezu quantitativ erhaltenen Verbindungen der Formel (IIa) sind über geläufige Reduktionsreaktionen in die Bisalkohole (IIb) überführbar. Diese sind auch direkt durch Oxidation (siehe z.B. A. Belli et al., Synthesis 1980, 477) aus den Verbindungen der Formel (VI) erhältlich.

Gegebenenfalls kann das Halogenatom auf einer geeigneten Stufe, wie im folgenden für die Verbindungen der Formel (llla) beschrieben, gegen eine Boronsäure(ester)- oder Trialkylzinn-Gruppe ausgetauscht werden.

Die Herstellung entsprechender Perfluoralkylsulfonate kann beispielsweise durch Veresterung entsprechender Phenolfunktionen erfolgen.

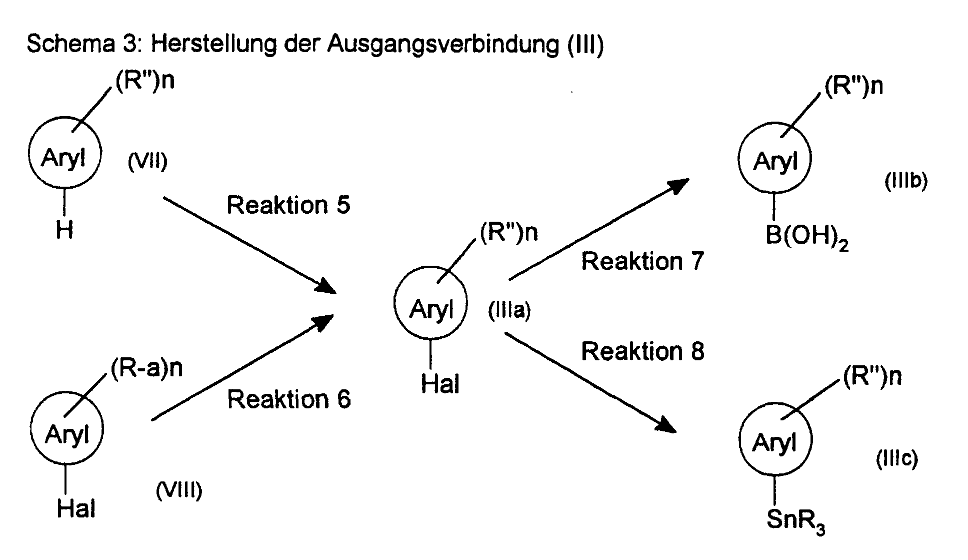

Schema 3 ist wie folgt zu erläutern: Die Verbindungen (VII) sind in der Regel käuflich zu erwerben (z. B. diverse Alkyl- und Dialkylaromaten, Alkoxyaromaten) oder einfach aus entsprechenden Vorstufen (z. B. Hydrochinon, Brenzcatechin, Naphthol), z.B. durch Alkylierung, herzustellen. Verbindung (VII) kann dann durch einfache Halogenierungsreaktionen (Reaktion 5) wie oben beschrieben in Verbindungen der Formel (IIIa) umgewandelt werden. Viele Verbindungen der Formel (VIII) sind wohlfeile Chemikalien (z. B. Bromphenol, Bromanilin), welche durch Reaktion 6 (z. B. Alkylierung von Phenolfunktionen) einfach in Verbindungen der Formel (IIIa) überführbar sind. Diese sind nun durch entsprechende Reagentien (z. B. Mg-Späne, n-BuLi, s-BuLi) zu metallieren und dann durch entsprechende weitere Umsetzung, z. B. mit Trialkylzinnchlorid, Borsäuretrialkylester, in die entsprechenden Verbindungen der Formel (IIIb) bzw. (IIIc) umwandelbar.

Damit wird gezeigt, daß die Ausgangsverbindungen (II) und (III) in der geforderten Variationsbreite einfach zugänglich sind.

Erfindungsgemäß werden die Ausgangsverbindungen (II) und (III) durch eine Kupplungsreaktion (Reaktion A in Schema 1) zu Zwischenprodukten der Formel (IV) umgesetzt.

Dazu werden die Verbindungen der Formeln (II) und (III) in einem inerten Lösungsmittel bei einer Temperatur im Bereich von 0°C bis 200°C in Gegenwart eines Palladiumkatalysators zur Reaktion gebracht.
Dabei enthält jeweils eine der Verbindungen, vorzugsweise die der Formel (II), eine Halogen- oder Perfluoralkylsulfonat-Gruppe, die andere eine Boronsäure(ester)-Gruppe (IIIb) oder eine Trialkylzinn-Gruppe (Illc).

Zur Durchführung der erfindungsgemäßen Reaktion A mit Boronsäure(ester)n der Formel (IIIb), Variante Aa, Suzuki-Kupplung, werden die aromatische Borverbindung, die aromatische Halogenverbindung bzw. das Perfluoralkylsulfonat, eine Base und katalytische Mengen des Palladiumkatalysators in Wasser oder in ein oder mehrere inerte organische Lösungsmittel oder vorzugsweise in eine Mischung aus Wasser und einem oder mehreren inerten organischen Lösungsmitteln gegeben und bei einer Temperatur von 0°C bis 200°C, bevorzugt bei 30°C bis 170°C, besonders bevorzugt bei 50°C bis 150°C, insbesonders bevorzugt bei 60°C bis 120°C für einen Zeitraum von 1 h bis 100 h, bevorzugt 5 h bis 70 h, besonders bevorzugt 5 h bis 50 h, umgesetzt, z.B. gerührt. Das Rohprodukt kann nach dem Fachmann bekannten und dem jeweiligen Produkt angemessenen Methoden, z.B. durch Umkristaliisation, Destillation, Sublimation, Zonenschmelzen, Schmelzkristallisation oder Chromatographie, aufgereinigt werden.

Für das erfindungsgemäße Verfahren geeignete organische Lösungsmittel sind beispielsweise Ether, z. B. Diethylether, Dimethoxymethan, Diethylenglykoldimethylether, Tetrahydrofuran, Dioxan, Dioxolan, Diisopropylether, tert.-Butylmethylether, Kohlenwasserstoffe, z. B. Hexan, iso-Hexan, Heptan, Cyclohexan,Toluol, Xylol, Alkohole, z. B. Methanol, Ethanol, 1-Propanol, 2-Propanol, Ethylenglykol, 1-Butanol, 2-Butanol, tert.-Butanol, Ketone, z. B. Aceton, Ethylmethylketon, iso-Butylmethylketon, Amide, z.B. Dimethylformamid, Dimethylacetamid, N-Methylpyrrolidon, Nitrile, z.B. Acetonitril, Propionitril, Butyronitril, und Mischungen derselben.

Bevorzugte organische Lösungsmittel sind Ether, wie Dimethoxyethan, Diethylenglykoldimethylether, Tetrahydrofuran, Dioxan, Diisopropylether, Kohlenwasserstoffe, wie Hexan, Heptan, Cyclohexan, Toluol, Xylol, Alkohole, wie Methanol, Ethanol, 1-Propanol, 2-Propanol, 1-Butanol, 2-Butanol, tert.-Butanol, Ethylenglykol, Ketone, wie Ethylmethylketon, iso-Butylmethylketon, Amide, wie Dimethylformamid, Dimethylacetamid, N-Methylpyrrolidon, und Mischungen derselben.

Besonders bevorzugte Lösungsmittel sind Ether, z. B. Dimethoxyethan, Tetrahydrofuran, Kohlenwasserstoffe, z. B. Cyclohexan, Toluol, Xylol, Alkohole, z. B. Ethanol, 1-Propanol, 2-Propanol, 1-Butanol, tert.-Butanol und Mischungen derselben.

In einer besonders bevorzugten Variante werden Wasser und ein oder mehrere in Wasser unlösliche Lösungsmittel eingesetzt.
Beispiele sind Mischungen aus Wasser und Toluol sowie Wasser, Toluol und Tetrahydrofuran.

Basen, die bei dem erfindungsgemäßen Verfahren vorzugsweise Verwendung finden sind Alkali- und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate, sowie primäre, sekundäre und tertiäre Amine.

Besonders bevorzugt sind Alkali- und Erdalkalimetallhydroxide, Alkali- und Erdalkalimetallcarbonate und Alkalimetallhydrogencarbonate. Insbesondere bevorzugt sind Alkalimetallhydroxide, wie Natriumhydroxid und Kaliumhydroxid, sowie Alkalimetallcarbonate und Alkalimetallhydrogencarbonate, wie Lithiumcarbonat, Natriumcarbonat und Kaliumcarbonat.

Die Base wird bevorzugt mit einem Anteil von 100 bis 1000 Mol-%, besonders bevorzugt 100 bis 500 Mol-%, ganz besonders bevorzugt 150 bis 400 Mol-%, insbesondere 180 bis 250 Mol-%, bezogen auf die aromatische Borverbindung, eingesetzt.

Der Palladiumkatalysator enthält Palladiummetail oder eine Palladium (0) oder (II) Verbindung und einen Komplexliganden, vorzugsweise einen Phosphanliganden.

Die beiden Komponenten können eine Verbindung bilden, z.B. das besonders bevorzugte Pd(PPh₃)₄, oder getrennt eingesetzt werden.

Als Palladiumkomponente eignen sich beispielsweise Palladiumverbindungen, wie Palladiumketonate, Palladiumacetylacetonate, Nitrilpalladiumhalogenide, Olefinpalladiumhalogenide, Palladiumhalogenide, Allylpalladiumhalogenide und Palladiumbiscarboxylate, bevorzugt Palladiumketonate, Palladiumacetylacetonate, bis-η²-Olefinpalladiumdihalogenide, Palladium(II)halogenide, η-³-Allylpalladiumhalogenid Dimere und Palladiumbiscarboxylate, ganz besonders bevorzugt Bis(dibenzylidenaceton)palladium(0) [Pd(dba)₂)], Pd(dba)₂•CHCl₃, Palladiumbisacetylacetonat, Bis(benzonitril)palladiumdichlorid, PdCl₂, Na₂PdCl₄, Dichlorobis(dimethylsulfoxid)palladium(II), Bis(acetonitril)palladiumdichlorid, Palladium-(II)-acetat, Palladium-(II)-propionat, Palladium-(II)-butanoat und (1c,5c-Cyclooctadien)palladiumdichlorid.

Ebenso als Katalysator dienen kann Palladium in metallischer Form, im folgenden nur Palladium genannt, vorzugsweise Palladium in pulverisierter Form oder auf einem Trägermaterial, z.B. Palladium auf Aktivkohle, Palladium auf Aluminiumoxid, Palladium auf Bariumcarbonat, Palladium auf Bariumsulfat, Palladium auf Aluminiumsilikaten, wie Montmorillonit, Palladium auf SiO₂ und Palladium auf Calciumcarbonat, jeweils mit einem Palladiumgehalt von 0,5 bis 10 Gew.-%. Besonders bevorzugt sind Palladium in pulverisierter Form, Palladium auf Aktivkohle, Palladium auf Barium- und/oder Calciumcarbonat und Palladium auf Bariumsulfat, jeweils mit einem Palladiumgehalt von 0,5 bis 10 Gew.-% insbesondere bevorzugt ist Palladium auf Aktivkohle mit einem Palladiumgehalt von 5 oder 10 Gew.-%

Der Palladiumkatalysator wird bei dem erfindungsgemäßen Verfahren mit einem Anteil von 0,01 bis 10 Mol-%, bevorzugt 0,05 bis 5 Mol-%, besonders bevorzugt 0,1 bis 3 Mol-%, insbesondere bevorzugt 0,1 bis 1,5 Mol-%, bezogen auf die aromatische Halogenverbindung oder das Perfluoralkylsulfonat, eingesetzt.

Für das erfindungsgemäße Verfahren geeignete Komplexliganden sind beispielsweise Phosphane, wie Trialkylphosphane, Tricycloalkylphosphane, Triarylphosphane, wobei die drei Substituenten am Phosphor gleich oder verschieden, chiral oder achiral sein können und wobei einer oder mehrere der Liganden die Phosphorgruppen mehrerer Phosphane verknüpfen können und wobei ein Teil dieser Verknüpfung auch ein oder mehrere Metallatome sein können. Beispiele für im Rahmen des erfindungsgemäßen Verfahrens verwendbare Phosphane sind Trimethylphosphan, Tributylphosphan, Tricyclohexylphosphan, Triphenylphosphan, Tritolylphosphan, Tris-(4-dimethylaminophenyl)-phosphan, Bis(diphenylphosphano)methan, 1,2-Bis(diphenylphosphano)ethan, 1,3-Bis(diphenylphosphano)propan und 1,1'-Bis(diphenylphosphano)-ferrocen. Weitere geeignete Liganden sind beispielsweise Diketone, z. B. Acetylaceton und Octafluoracetylaceton und tert. Amine, z. B. Trimethylamin, Triethylamin, Tri-n-propylamin und Triisopropylamin.
Bevorzugte Komplexliganden sind Phosphane und Diketone, besonders bevorzugt sind Phosphane.

Ganz besonders bevorzugte Komplexliganden sind Triphenylphosphan, 1,2-Bis(diphenylphosphano)ethan, 1,3-Bis(diphenylphosphano)propan und 1,1'-Bis(diphenylphosphano)-ferrocen, insbesondere Triphenylphosphan.

Für das erfindungsgemäße Verfahren weiterhin geeignet sind wasserlösliche Komplexliganden, die beispielsweise Sulfonsäuresalz- und/oder Sulfonsäurereste und/oder Carbonsäuresalz- und/oder Carbonsäurereste und/oder Phosphonsäuresalz und/oder Phosphonsäurereste und/oder Phosphoniumgruppen und/oder Peralkylammoniumgruppen und/oder Hydroxygruppen und/oder Polyethergruppen mit geeigneter Kettenlänge enthalten.

Bevorzugte Klassen von wasserlöslichen Komplexliganden sind mit den obigen Gruppen substituierte Phosphane, wie Trialkylphosphane, Tricycloalkylphosphane, Triarylphosphane, Dialkylarylphosphane, Alkyldiarylphosphane und Heteroarylphosphane wie Tripyridylphosphan und Trifurylphosphan, wobei die drei Substituenten am Phosphor gleich oder verschieden, chiral oder achiral sein können und wobei einer oder mehrere der Liganden die Phosphorgruppen mehrerer Phosphane verknüpfen können und wobei ein Teil dieser Verknüpfung auch ein oder mehrere Metallatome sein können, Phosphite, Phosphinigsäureester und Phosphonigsäureester, Phosphole, Dibenzophosphole und Phosphoratome enthaltende cyclische bzw. oligo- und polycyclische Verbindungen.

Der Komplexligand wird im allgemeinen mit einem Anteil von 0,1 bis 20 Mol %, bevorzugt 0,2 bis 15 Mol %, besonders bevorzugt 0,5 bis 10 Mol %, insbesonders bevorzugt 1 bis 6 Mol %, bezogen auf die aromatische Halogenverbindung oder das Perfluoralkylsulfonat, eingesetzt.
Es können gegebenenfalls auch Mischungen zweier oder mehrerer verschiedener Komplexliganden eingesetzt werden.

Das erfindungsgemäß eingesetzte Boronsäurederivat kann ganz oder teilweise als Anhydrid vorliegen.

Vorteilhafte Ausführungsformen von Teilen des erfindungsgemäßen Verfahrens der Variante Aa sind z.B. in WO-A-94/101 05, EP-A-679 619, EP-A 694 530 und PCT/EP 96/03154 beschrieben, auf die hiermit ausdrücklich Bezug genommen wird. Sie gelten durch Zitat als Bestandteil der Beschreibung dieser Anmeldung.

Bei der Variante Ab, auch Stille-Kupplung genannt, wird eine aromatische Zinnverbindung, vorzugsweise der Formel (lllc), mit einer aromatischen Halogenverbindung oder einen aromatischen Perfluoralkylsulfonat vorzugsweise der Formel (II), bei einer Temperatur im Bereich von 0°C bis 200°C in einem inerten organischen Lösungsmittel in Gegenwart eines Palladiumkatalysators umgesetzt.

Ein Überblick über diese Reaktion findet sich z.B. bei J.K. Stille, Angew. Chemie Int. Ed. Engl. 1986, 25, 508.

Zur Durchführung des Verfahrens werden bevorzugt die aromatische Zinnverbindung und die aromatische Halogenverbindung bzw. das Perfluoralkylsulfonat in ein oder mehrere inerte organische Lösungsmittel gegeben und bei einer Temperatur von 0°C bis 200°C, bevorzugt bei 30°C bis 170°C, besonders bevorzugt bei 50°C bis 150°C, insbesondere bevorzugt bei 60°C bis 120°C für einen Zeitraum von 1 h bis 100 h, bevorzugt 5 h bis 70 h, besonders bevorzugt 5 h bis 50 h, umgesetzt, z.B. gerührt. Nach beendeter Umsetzung wird der als Feststoff anfallende Pd-Katalysator beispielsweise durch Filtration abgetrennt und das Rohprodukt vom Lösungsmittel bzw. den Lösungsmitteln befreit. Anschließend kann nach dem Fachmann bekannten und dem jeweiligen Produkt angemessene Methoden, z.B. durch Umkristallisation, Destillation, Sublimation, Zonenschmelzen, Schmelz-kristallisation oder Chromatographie, weiter aufgereinigt werden.

Geeignete organische Lösungsmittel sind beispielsweise Ether, z.B. Diethylether, Dimethoxymethan, Diethylenglykoldimethylether, Tetrahydrofuran, Dioxan, Dioxolan, Diisopropylether, tert.-Butylmethylether, Kohlenwasserstoff, z.B. Hexan, iso-Hexan, Heptan, Cyclohexan, Benzol, Toluol, Xylol, Alkohole, z.B. Methanol, Ethanol, 1-Propanol, 2-Propanol, Ethylenglykol, 1-Butanol, 2-Butanol, tert.-Butanol, Ketone, z.B. Aceton, Ethyl-methylketon, iso-Butylmethylketon, Amide, z.B.

Dimethylformamid (DMF), Dimethylacetamid, N-Methylpyrrolidon, Nitrile, z.B. Acetonitril, Propionitril, Butyronitril und Mischungen derselben.

Bevorzugte organische Lösungsmittel sind Ether, wie Dimethoxyethan, Diethylenglykoldimethylether, Tetrahydrofuran, Dioxan, Diisopropylether, Kohlenwasserstoffe, wie Hexan, Heptan, Cyclohexan, Benzol, Toluol, Xylol, Alkohole, wie Methanol, Ethanol, 1-Propanol, 2-Propanol, 1-Butanol, 2-Butanol, tert.-Butanol, Ethylenglykol, Ketone, wie Ethylmethylketon, oder Amide, wie DMF.

Besonders bevorzugte Lösungsmittel sind Amide, ganz besonders bevorzugt ist DMF.

Der Palladiumkatalysator enthält Palladiummetall oder eine Palladium (0) oder (II) Verbindung und einen Komplexliganden, vorzugsweise einen Phosphanliganden.

Die beiden Komponenten können eine Verbindung bilden, z.B. Pd(PPh₃)₄, oder getrennt eingesetzt werden.

Als Palladiumkomponente eignen sich beispielsweise Palladiumverbindungen, wie Palladiumketonate, Palladiumacetylacetonate, Nitrilpalladiumhalogenide, Olefinpalladiumhalogenide, Palladiumhalogenide, Allylpalladiumhalogenide und Palladiumbiscarboxylate, bevorzugt Palladiumketonate, Palladiumacetylacetonate, bis-η²-Olefinpalladiumdihalogenide, Palladium(ll)halogenide, η-³-Allylpalladiumhalogenid Dimere und Palladiumbiscarboxylate, ganz besonders bevorzugt Bis(dibenzylidenaceton)palladium(0) [Pd(dba)₂)], Pd(dba)₂•CHCl₃, Palladiumbisacetylacetonat, Bis(benzonitril)palladiumdichlorid, PdCl₂, Na₂PdCl₄, Dichlorobis(dimethylsulfoxid)palladium(ll), Bis(acetonitril)palladiumdichlorid, Palladium-(ll)-acetat, Palladium-(II)-propionat, Palladium-(ll)-butanoat und (1c,5c-Cyclooctadien)palladiumdichlorid.

Der Palladiumkatalysator wird bei dieser Variante des erfindungsgemäßen Verfahrens im allgemeinen mit einem Anteil von 0,01 bis 10 Mol-%, bevorzugt 0,05 bis 5 Mol-%, besonders bevorzugt 0,1 bis 3 Mol-%, insbesondere bevorzugt 0,1 bis 1,5 Mol-%, bezogen auf die aromatische Halogenverbindung oder das Perfluoralkylsulfonat, eingesetzt.

Geeignete Liganden sind beispielsweise Phosphane, wie Trialkylphosphane, Tricycloalkylphosphane, Triarylphosphane, wobei die drei Substituenten am Phosphor gleich oder verschieden, chiral oder achiral sein können und wobei einer oder mehrere der Liganden die Phosphorgruppen mehrerer Phosphane verknüpfen können und wobei ein Teil dieser Verknüpfung auch ein oder mehrere Metallatome sein können.

Der Ligand wird bei dieser Variante des erfindungsgemäßen Verfahrens im allgemeinen mit einem Anteil von 0,1 bis 20 Mol-%, bevorzugt 0,2 bis 15 Mol-%, besonders bevorzugt 0,5 bis 10 Mol-%, insbesonders bevorzugt 1 bis 6 Mol-%, bezogen auf die aromatische Halogenverbindung oder das Perfluoralkylsulfonat, eingesetzt.

### Reaktion B

Falls die Gruppe X' in dem Zwischenprodukt (IV) -COOR, ist wird zum Bisalkohol, X' = CH₂OH, reduziert.

Die Reduktion kann nach bekannten, dem Fachmann geläufigen Methoden erfolgen, wie sie beispielsweise in Houben-Weyl, 4. Aufl, Bd. 6, 16, Kap. VIII, Georg-Thieme-Verlag, Stuttgart 1984, beschrieben sind.
Bevorzugte Ausführungsformen sind
a) Umsetzung mit Li-AlH₄ oder Diisobutylaluminiumhydrid (DIBAL-H) in Tetrahydrofuran (THF) oder Toluol, wie beispielsweise in Organikum (s.o), S. 612 ff. beschrieben;
b) Umsetzung mit Borhydriden, wie BH₃, wie beispielsweise in Houben-Weyl, 4. Aufl, Bd. 6, 16, Kap. VIII, S. 211-219, Georg-Thieme-Verlag, Stuttgart 1984, beschrieben;
c) Umsetzung mit Wasserstoff in Gegenwart eines Katalysators, wie beispielsweise in Houben-Weyl, 4. Aufl, Bd. 6, 16, Kap. VIII, S. 110 f, Georg-Thieme-Verlag, Stuttgart 1984 beschrieben, und
d) Reaktion mit Natrium oder Natriumhydrid.

Besonders bevorzugt ist die Reduktion mit LiAlH₄ oder DIBAL-H.

### Reaktion C a

Die aus den Reaktionen A oder B erhaltenen Bisalkohole der Formel (IV) (X = CH₂OH) können durch selektive Oxidation in erfindungsgemäße Bisaldehyde der Formel (1) überführt werden.
Eine solche Oxidation kann nach an sich bekannten, dem Fachmann geläufigen Verfahren durchgeführt werden, wie sie beispielsweise in R.C. Laroch, Comprehensive Organic Transformations, VCH, 1989, S 604-614 und der dort zitierten Literatur beschrieben sind.

Bevorzugt sind:
a) die Oxidation mit Dimethylsulfoxid / Oxalylchlorid (Swern-Oxidation), wie sie beispielsweise bei A.J. Mancoso, D. Swern, Synthesis 1981, 165 beschrieben ist, und
b) die Oxidation mit Pyridiniumchlorochromat (PCC) oder Pyridiniumdichromat, wie sie beispielsweise in Houben-Weyl, 4. Auflage, Band E3, S. 291-296, Georg-Thieme Verlag, Stuttgart, 1983 beschrieben ist.

Die so erhaltenen Aldehyde lassen sich für Polymerisationsreaktionen, z.B. nach Wittig/Horner oder Knoevenagel, einsetzen.

### Reaktion C b

Erfindungsgemäß können die OH-Gruppen in den Bisalkoholen der Formel (IV) durch nucleophile Substitution gegen Halogen oder Pseudohalogen ausgetauscht werden.

Zur Herstellung von Chloriden und Bromiden ist es bevorzugt, den entsprechenden Bisalkohol mit HCI bzw. HBr, beispielsweise in Eisessig, umzusetzen (siehe z.B. Houben-Weyl, Band 5/4, S. 385 ff, 1960) oder mit Thionylchlorid bzw. -bromid, gegebenenfalls in Gegenwart eines Katalysators, zur Reaktion zu bringen (siehe z.B. Houben-Weyl, Band 5/1b, S. 862 ff., 1962).

Chloride lassen sich auch bevorzugt durch Reaktion mit Phosgen (siehe z.B. Houben-Weyl, Band V, 3, S. 952 ff, 1962), Bromide durch Reaktion mit PBr₃ herstellen.

lodide lassen sich vorzugweise durch Reaktion mit Phosphor/lod nach A.I. Vogel (siehe z.B. Houben-Weyl, Band V, 4, s. 615 f., 1969) herstellen.

Die Aufarbeitung erfolgt in allen Fällen in einfacher Weise nach bekannten, dem Fachmann geläufigen Methoden. Die erhaltenen Verbindungen der Formel (I) können für Polymerisationsreaktionen, beispielsweise Dehydrohalogenierungen oder Knoevenagelkondensationen (Z = CN), vorteilhaft eingesetzt werden.

### Reaktion D

Die Umwandlung von Halogenverbindungen der Formel (Ib) in Bis(diphenylphosphanoxide) bzw. Bis(phosphonsäureester) der Formel (Ic) ist beispielsweise unter Anwendung der Michaelis-Arbusov-Reaktion aus den entsprechenden Bis(halogenmethyl)-Verbindungen mit Diphenylphosphinigsäure-ethylester (C₆H₅)P-O-C₂H₅ bzw. mit Triethylphosphit leicht möglich. Bisphosphoniumsalze sind ebenfalls einfach durch Umsetzung der Halogenide mit bspw. Triarylphosphanen erhältlich.

Die so erhaltenen Verbindungen können für Polymerisationsreaktionen nach Wittig/Horner eingesetzt werden.

Produkte des erfindungsgemäßen Verfahrens sind polymerisierbare Biaryle der Formel (1), 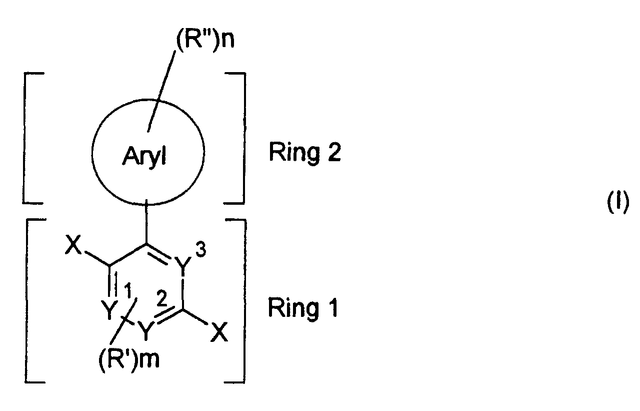 wobei die Symbole und Indizes die oben angegebene Bedeutungen haben.

Bevorzugt sind Verbindungen der Formel (I) bei denen die Symbole und Indizes folgende Bedeutungen haben:
- X: -CH₂Z, CHO;
- Z: Cl, Br, CN, PO(OR¹)₂, PO(R²)₂, P(R³)₃^{⊕}A^{⊖};
- Y¹, Y², Y³: CH;
- Aryl: Phenyl, 1- bzw. 2-Naphthyl, 1-, 2- bzw. 9-Anthracenyl, 2-, 3- bzw. 4-Pyridinyl, 2-, 4- bzw. 5-Pyrimidinyl, 2-Pyrazinyl, 3- bzw. 4-Pyridazinyl, 2-, 3-, 4-, 5-, 6-, 7- bzw. 8-Chinolin, 2- bzw. 3-Thiophenyl, 2- bzw. 3-Pyrrolyl, 2- bzw. 3-Furanyl und 2-(1,3,4-Oxadiazol)yl;
- R': gleich oder verschieden geradkettige oder verzweigte Alkoxygruppe mit 1 bis 12 C-Atomen;
- R": gleich oder verschieden geradkettige oder verzweigte Alkyl- oder Alkoxygruppe mit 1 bis 12 C-Atomen;
- m: 0, 1, besonders bevorzugt 0;
- n: 1, 2, 3, besonders bevorzugt 1, 2.

Besonders bevorzugt sind Verbindungen, in denen Ring 2 Phenyl, 1-Naphthyl, 2-Naphthyl oder 9-Anthracenyl ist.

Des weiteren sind in Ring 2 folgende Substitutionsmuster bevorzugt: 2-, 3- bzw. 4-Alkyl(oxy)phenyl, 2,3-, 2,4-, 2,5-, 2,6-, 3,4- bzw. 3,5-Dialkyl(oxy)phenyl, 2,3,4,-, 2,3,5-, 2,3,6-, 2,4,5, 2,4,6- bzw. 3,4,5-Trialkyl(oxy)phenyl, 2-, 3-, 4-, 5-, 6-, 7- bzw. 8-Alkyl(oxy)-1-naphthyl, 1-, 3-, 4-, 5-, 6-, 7- bzw. 8-Alkyl(oxy)-2-naphthyl und 10-Alkyl(oxy)-9-anthracenyl.

Bevorzugte Ausgangsverbindungen der Formeln (II) und (III) ergeben sich in eindeutiger Weise aus den Bevorzugungen der Endprodukte.

Die polymerisierbaren Biaryle der Formel (I) sind neu und als Zwischenprodukte zur Herstellung neuer Polymere mit besonderer Eignung als Elektrolumineszenzmaterialien geeignet.

Sie sind daher ebenfalls Gegenstand der Erfindung.

Weiterhin Gegenstand der Erfindung ist die Verwendung von polymerisierbaren Biarylen der Formel (I) zur Herstellung von Polymeren, die vorzugsweise als Elektrolumineszenzmaterialien eingesetzt werden.

In der vorliegenden Anmeldung sind verschiedene Dokumente zitiert, beispielsweise um das technische Umfeld der Erfindung zu illustrieren. Auf alle diese Dokumente wird hiermit ausdrücklich Bezug genommen, sie gelten durch Zitat als Bestandteil der vorliegenden Anmeldung.
Auf den Inhalt der deutschen Patentanmeldung 196 51 439.8, deren Priorität die vorliegenden Anmeldung beansprucht, sowie auf die Zusammenfassung der vorliegenden Anmeldung wird hiermit ausdrücklich Bezug genommen, sie gelten durch Zitat als Bestandteil der vorliegenden Anmeldung.

Die Erfindung wird durch die Beispiele näher erläutert, ohne sie dadurch einschränken zu wollen.

### A. Synthese von Verbindungen der Formel (II)

### Beispiel A1: Synthese von 2-Bromterephthalsäurediethylester und 2-Brom-1,4-bis(hydroxymethyl)benzol:

a) Synthese von 2-Brom-p-xylol:
   p-Xylol (934,4 g; 8,8 mol) wurde mit Fe-Pulver (16 g) vorgelegt, und ca. 20 ml Brom langsam zugetropft. Der Beginn der Reaktion (Dauer ca. 10 min.) war durch Gasentwicklung beobachtbar. Nachdem die Reaktion begonnen hatte, wurde das restliche Brom (gesamt: 1278,4 g; 8,0 mol) bei RT, mit Wasserbadkühiung, zugetropft (4 h). Es wurde 2 h bei RT nachgerührt. Die leicht braun gefärbte Reaktionslösung wurde abfiltriert und zuerst mit Wasser, dann mit 480 ml gesättigter, wäßriger Na₂SO₃-Lösung ausgerührt, anschließend noch einmal mit verdünnter, wäßriger NaOH und zweimal mit H₂O ausgeschüttelt; die organische Phase (wasserklar) wurde mit MgSO₄ getrocknet, filtriert und durch zweifache Vakuumdestillation (Membranpumpe / Ölbad ca. 100-120 °C /60 cm Kolonne) gereinigt.
   Produkt (Sdp. ca. 85-89°C bei 13 - 9 mbar; Ölbad = 120-155°C): 1234,1 g (83,4 %)
   ¹H NMR (400 MHz; CDCl₃): δ [ppm] = 7.33 (dd; 1 H; J₁ = 2, J₂ = 0.7 Hz; H-3), 7.06 (d (br); 1 H; J₁ = 8 Hz; H-6), 6.97 (dd; 1 H; J₁ = 8, J₂ = 2 Hz; H-5), 2.33 und 2.26 (jeweils: s (br); 3 H; Me).
b) Synthese von 2-Bromterephthalsäure:
   In einem 1L-Hastelloy-C22-Autoklaven wurde eine Lösung von Brom-p-xylol (92.5 g, 0.5 mol), Kobaltacetattetrahydrat (0.62 g, 2.5 mmol), Manganacetattetrahydrat (0.61 g, 2.5 mmol), Bromwasserstoff (0.4 g, 5.0 mmol) und Kaliumacetat (0.98 g, 10 mmol) in 350 g Eisessig vorgelegt.
   Die Lösung wurde unter Rühren in einer Stickstoffatmosphäre (18 bar) erhitzt. Bei 154°C wurde Pressluft durch die Lösung geleitet (18 bar; Lufteinspeisung ca. 180 Liter pro Stunde). Die Reaktion begann sofort. Die Reaktionstemperatur wurde durch Außenkühiung bei ca. 165°C gehalten. Nach einer Stunde ist die exotherme Reaktion beendet, der Reaktorinhalt wurde erneut mit Stickstoff überlagert und auf 100°C abgekühlt. Die bei dieser Temperatur entnommene Suspension wurde unter Rühren auf 20°C abgekühlt und das Kristallisat abfiltriert.
   Nach dreimaligem Waschen mit jeweils 50 ml Eisessig wurde das farblose Produkt bei 50 °C und 65 mbar getrocknet. Produkt: farbloses, mikrokristallines Pulver, 102.2 g (83.7 % d. Th.), Schmelzpunkt 302°C.
   ¹H NMR (400 MHz; d₆-DMSO): δ [ppm] = 13.7 (br; 2 H; CO₂H), 8.18 (d; 1 H; J₁ = 2 Hz; H-3), 8.02 (dd; 1 H; J₁ = 8, J₂ = 2 Hz; H-5), 7.85 (d; 1 H; J₁ = 8 Hz; H-6).
c1) Erste Syntheseroute zu 2-Bromterephthalsäurediethylester:
   2-Bromterephthalsäure (122.52 g; 0,5 mol) wurde in Ethanol (138 g; 3 mol) und Tetrachlorkohlenstoff (150 ml) suspendiert, 15 ml Schwefelsäure wurden mit einer Pipette zugegeben und die Mischung unter kräftigem Rühren für 5 Tage am Rückfluß gekocht. Die Suspension wandelte sich innerhalb von ca. 24 Stunden in eine klare Lösung um, die Reaktion war aber (DC-Kontrolle) erst nach 5 Tagen beendet. Anschließend wurden die Phasen getrennt und die organische Phase mit H₂O, sowie wäßriger NaHCO₃-Lösung ausgeschüttelt, wobei die überstehende wäßrige Phase leicht alkalisch wurde. Nach nochmaligem Ausschütteln mit H₂O wurde die organische Phase über Na₂SO₄ getrocknet und das Lösungsmittel abgezogen. Das gewünschte Produkt fiel ohne weitere Reinigung als gelbliches, schwach viskoses Öl nahezu sauber an (97- 98 %) an: 118 g (78 %), d 1,38 kg/dm³. Zur weiteren Reinigung war fraktionierte Vakuumdestillation geeignet. Es wurde 99.9 %iges Produkt (¹H-NMR) bei 1.1 mbar und 142°C erhalten.
   ¹H NMR (400 MHz; CDCl₃): δ [ppm] = 8.30 (d; 1 H; J₁ = 1.7 Hz; H-3), 8.01 (dd; 1 H; J₁ = 8, J₂ = 1.7 Hz; H-5), 7.79 (d; 1 H; J₁ = 8 Hz; H-6), 4.43, 4.41 (jeweils: q; 2 H; J = 7.1 Hz; O-CH₂), 1.42, 1.41 (jeweils: t; 3 H; J = 7.1 Hz; CH₃).
c2) Zweite Syntheseroute zu 2-Bromterephthalsäurediethylester:
   Bromterephthalsäure (500 g, 2.04 mol) wurde unter Schutzgas vorgelegt, bei Raumtemperatur unter Rühren mit SOCl₂ (728 g, 446 ml, 6.12 mol) vermischt und mit 3 Tropfen DMF (N,N-Dimethylformamid) versetzt. Die Mischung war auch nach Ende der 90-minütigen Zugabe breiartig und damit schlecht rührbar. Anschließend wurde auf 60°C Innentemperatur aufgeheizt und bei dieser Temperatur 4 Tage gerührt; danach lag eine klare Lösung vor. Der Ansatz wurde vom überschüssigem Thionylchlorid befreit, indem man 2x je 100 ml Toluol zusetzte und das Thionylchlorid/Toluol-Gemisch bei Normaldruck jeweils abdestillierte (140° Badtem.). Das resultierende flüssige Säurechlorid wurde unter Wasserbadkühlung innerhalb ca. 50 min mit absolutem Ethanol (460 g, 583 ml, 10 mol) versetzt (Temperaturanstieg auf 45°) und über Nacht unter Rückfluß erhitzt. Verunreinigungen wurden abfiltiert und das Lösungsmittel abgezogen. Das honigfarbene, leicht viskose Produkt wurde im Ölpumpenvakuum getrocknet: 612.7 g (+99 % d. Th.); ca. 97%ige Reinheit (¹H-NMR).
   NMR: analog zu c1). Weitere Reinigung analog c1.
c3) Dritte Syntheseroute zu 2-Bromterephthalsäuredieethylester:
   Bromterephthalsäure (49 g, 0.2 mol) wurde mit EtOH (184 g, 233 ml, 4.0 mol) unter Schutzgas vorgelegt und dann bei RT unter Rühren mit H₂SO₄ (1 ml) versetzt. Anschließend wurde am Rückfluß (78°C) gekocht. Die anfangs weiße Suspension lag nach 20 Minuten als klare Lösung vor. Das Ethanol wurde abdestilliert, bis die Innentemperatur 110°C erreicht hatte. Anschließend wurde erneut frisches Ethanol (200 ml) zugesetzt und die Prozedur von vorne begonnen. Dieses Verfahren wurde insgesamt fünfmal wiederholt, dann war die Reaktion laut DC beendet. Am Ende der Reaktion wurde restliches Ethanol möglichst vollständig abdestilliert, der Reaktionsansatz mit etwas Ethylacetat versetzt und durch Schütteln zunächst mit wäßriger NaHCO₃-Lösung und schließlich mit H₂O neutral gewaschen. Das organische Lösungsmittel wurde abgezogen und das ölige Produkt an der Ölpumpe getrocknet: 56.6 g (94%), Reinheit (It. ¹H-NMR) ca. 97%. Weitere Reinigung analog c1.
   NMR: analog zu c1).
d) Synthese von 2-Brom-1,4-bishydroxymethylbenzol:
   1. Stufe:
      122.82 g (0.50 mol) Bromterephthalsäure wurden vorgelegt und unter N₂ mit 3 Tropfen DMF versetzt. 110 ml (1.5 mol) SOCl₂ wurden bei Raumtemperatur zuerst langsam und dann schnell zugetropft (Suspension etwas besser rührbar, aber noch immer breiförmig; Dauer: ca. 70 min). Die Suspension wurde vorsichtig erhitzt und bei 55 °C Innentemperatur 7 Std. gerührt. Nach Stehen über Nacht bei Raumtemperatur wurde der Ansatz destillativ von überschüssigem Thionylchlorid befreit. Dazu wurde der Ansatz 2x mit je 50 ml Hexan versetzt und das Thionylchlorid-Hexan-Gemisch bei Normaldruck abdestilliert. Zum Schluß wurde noch für ca. 30 min. ein Vakuum von 100 mbar angelegt.
   2. Stufe:
      23.1 g (0.6 mol) LiAlH₄ wurden unter N₂ mit 500 ml THF abs. versetzt. Zur grauen Suspension wurde bei Raumtemperatur eine Lösung aus der 1. Stufe (ca. 90 ml) in 200 ml THF abs. zugetropft (Dauer: ca. 3 Std.). Der Ansatz wurde nun zum Rückfluß erhitzt und 5.5 Stunden gerührt. Nach Abkühlen auf Raumtemperatur wurde die beige Suspension im Eisbad weiter abgekühlt. 46 g Eiswasser wurden vorsichtig zugetropft (Dauer: ca. 1 Std.). Nach Zugabe von weiteren 50 ml H₂O wurden 100 ml 1 N wäßrige H₂SO₄ und dann 90 ml ½ konzentrierter wäßriger H₂SO₄ zugetropft. Man erhielt 2 Phasen: obere: gelb, homogen; untere: graue Suspension. Die Phasen wurden getrennt und die untere, graue Phase wurde 2x mit je 200 ml Ethylacetat extrahiert. Die vereinigten organischen Phasen wurden 4x mit je 200 ml H₂O extrahiert und schließlich zur Trockene eingeengt. Man erhielt das Rohprodukt als beigen Feststoff (110 g), der durch Umkristallisation (H₂O/Ethanol = 2/1) weiter zu reinigen war. Produkt: farblose Nadeln (78 g; 72%), Schmelzpunkt: 106-108°C. ¹H NMR (400 MHz; d₆-Aceton): δ [ppm] = 7.55 (m; 2 H; H-3, H-6), 7.35 (dd; 1 H; J₁ = 8, J₂ = 1.9 Hz; H-5), 4.66, 4.62 (jeweils: d; 2 H; J = 5.9 Hz; CH₂-O), 4.37, 4.28 (jeweils: t; 1 H; J = 5.9 Hz; OH).

### Beispiel A2: Synthese von 2-Brom-5-methoxyterephthalsäurediethylester:

a) Synthese von 4-Brom-2,5-dimethylanisol
   Zu einer vorgelegten Mischung aus 2,5-Dimethylanisol (250 g, 1835 mmol) und Fe-Pulver (3.25 g) wurde unter Rühren tropfenweise Brom (291.5 g, 1835 mmol) zugesetzt. Der Beginn der Reaktion war durch Gasentwicklung sichtbar. Danach tropfte man das restliche Brom bei Raumtemperatur unter Wasserbadkühlung innerhalb 30-40 Minuten zu. Die Reaktionsmischung wurde ca. 4 Stunden weitergerührt. Anschließend wurde vom Fe-Pulver abgetrennt, wenig Chloroform zugesetzt und mit Wasser ausgeschüttelt, was zu einer Aufhellung der Lösung führte. Nach Schütteln mit 50 ml gesättigter wäßriger Na₂SO₃-Lösung war die Lösung vollends entfärbt. Man schüttelte noch einmal mit verdünnter wäßriger NaOH und zweimal mit H₂O und zog nach Trocknung das Lösemittel ab.
   Das Rohprodukt wurde im Vakuum fraktioniert destilliert.
   Das Produkt erhielt man als viskoses, farbloses Öl (Siedepunkt 68°C, 0.8 mbar): 285 g (72%)
   ¹H NMR (CDCl₃): δ [ppm] = 7.25 (s, 1 H, H-Aryl), 6.68 (s, 1 H, H-Aryl), 3.78 (s, 3 H, O-Me), 2.36, 2.14 (jeweils s, 3 + 3 H, CH₃).
b) Synthese von 2-Brom-5-methoxyterephthalsäure
   In einem 1-1-Autoklav (HC-22) mit Scheibenrührer, Rückflußkühler, Gaseinleitung und Gasauslaß wurde eine Lösung von Kobaltacetattetrahydrat (1.25 g, 5 mmol), Manganacetat-tetrahydrat (1.23 g), HBr (0.81 g), Natriumacetat (1.37 g) und 4-Brom-2,5-dimethylanisol (107.5 g, 0.5 mol) in 380 g Eisessig vorgelegt.
   Die Reaktionslösung wurde unter Stickstoffatmosphäre (17 bar) unter Rühren auf 150°C erhitzt. Bei dieser Temperatur wurde Luft (17 bar) durch die Lösung geleitet (180-200 l/h), worauf die exotherme Reaktion sofort ansprang. Die Reaktionstemperatur blieb durch Außenkühlung bei 150°C. Nach ca. 45 Minuten war die exotherme Reaktion beendet. Zur Ermöglichung einer Nachreaktion wurde bei 150°C für 30 min. ein Luft/Stickstoff-Gemisch (10% O₂) durchgeleitet. Danach wurde die Luftzufuhr abgebrochen und Stickstoff eingeleitet.
   Der Reaktorinhalt wurde unter Stickstoffatmosphäre auf 100°C abgekühlt, als Lösung in einen Kolben abgelassen und unter Rühren auf 20°C abgekühlt, wobei das Produkt auskristallisierte. Der farblose Kristallbrei wurde abgesaugt und viermal mit jeweils 40 g Eisessig gewaschen.
   Nach Trocknung erhielt man 96.2 g 2-Brom-5-methoxyterephthalsäure (70%).
   ¹H NMR (DMSO): δ [ppm] = 13.5 (br, 2 H, COOH), 7.87 (s, 1 H, H-Aryl), 7.42 (s, 1 H, H-Aryl), 3.88 (s, 3 H, O-Me).
c) Synthese von 2-Brom-5-methoxyterephthalsäurediethylester
   2-Brom-5-methoxyterephthalsäure (202.89 g, 738 mmol) wurde mit 500 ml EtOH unter Schutzgas vorgelegt und dann bei RT unter Rühren mit H₂SO₄ versetzt. Anschließend kochte man bei 78°C Innentemperatur am Rückfluß und destillierte EtOH ab, bis die Innentemperatur über 100 °C lag. Es wurde zunächst erneut Ethanol zugeführt, dieses dann wieder abdestilliert. Der Vorgang wurde solange wiederholt, bis laut DC nur noch der Diester vorhanden war. Schließlich wurde alles Ethanol abgezogen, das erhaltene Rohprodukt in Ethylacetat aufgenommen, mit wäßriger NaHCO₃-Lösung extrahiert und schließlich nach Phasentrennung und Trocknung erneut alles Lösungsmittel abgezogen. Der dabei erhaltene erstarrte Feststoff konnte, nach Zerkleinern, durch Rühren mit Hexan gereinigt werden.
   Man erhielt 190.4 g (78%) hellgelbe Kristalle.
   Schmelzpunkt: 61-63 °C
   ¹H NMR (CDCl₃): δ [ppm] = 8.00 (s, 1 H, H-Aryl), 7.34 (s,1 H, H-Aryl), 4.43 + 4.37 (jeweils q, 2 + 2 H, OCH₂, J = 7.5 Hz), 3.92 (s, 3 H, O-Me), 1.42 + 1.38 (jeweils t, 3 + 3 H, CH₃, J = 7.5 Hz).

### B. Synthese von Verbindungen der Formel (III)

### Beispiel B1: Synthese von 4-Hexyloxybenzolboronsäure:

a) Synthese von 4-Hexyloxybrombenzol:
   4-Bromphenol (173 g, 1 mol) wurde in ca. 500 ml frisch dest. THF unter Schutzgas gelöst und nach Durchleiten von Argon portionsweise mit NaH (33 g, (80%ig in Öl), 1.1 mol) versetzt. Dabei wurde die klare Lösung bei 20° Temperaturerhöhung trübgrau. Die Suspension wurde unter Schutzgasüberlagerung bei RT ca. 1 Stunde, gerührt. Hexylbromid (181 g; 149 ml; 1,1 mol) wurde im Tropftrichter kurz mit N₂ begast und unter Rühren innerhalb 25 Minuten zugegeben. Die immer noch graue Mischung wurde bei 75°C am Rückfluß gekocht. Nach 3 Tagen (die Suspension war mittlerweile heller) wurde das entstandene Salz abgesaugt und das Filtrat zur Vernichtung eventueller Reste von NaH mit 20 ml EtOH (keine Gasentwicklung) versetzt. Die gelbliche Lösung wurde eingeengt und aus der Lösung (trüb) das Produkt mittels
   fraktionierter Vakuumdestillation isoliert: Produkt: 95°C/1 mbar; 172.5 g (67 %); (d ∼ 1.17).
   ¹H NMR (400 MHz; CDCl₃): δ [ppm] = 7.35, 6.76 (AA'BB'; 4 H; H-aryl), 3.91 (t; 2 H; J = 7.5 Hz; O-CH₂), 1.77 (pseudo-quin; 2 H; J = 7.3 Hz; O-CH₂-CH₂), 1.45-1.25 (m; 6 H; H-alkyl), 0.91 (pseudo-t; 3 H; J = 7.7 Hz; CH₃).
b) Synthese von 4-Hexyloxybenzolboronsäure:
   Magnesiumspäne (1.89 g; 78 mmol) wurden in einer ausgeheizten, unter Argon stehenden Apparatur mit einem Kristall lod versetzt und mit getrocknetem THF überdeckt. Anschließend tropfte man in die ruhende Lösung einige Tropfen 4-Hexyloxybrombenzol zu. Die Grignardreaktion begann sehr schnell, anschließend tropfte man unter Rühren das 4-Hexyloxybrombenzol (Gesamtmenge: 20 g; 78 mmol) so zu, daß der Ansatz leicht siedete und verdünnte in der Zwischenzeit mit etwas THF (insgesamt ca. 100 ml). Es wurde für 3 Stunden am Rückfluß gekocht (nur noch wenige Magnesiumflitter in der Lösung), anschließend ließ man abkühlen. Die Grignardlösung wurde im Schutzgasgegenstrom in einen 250 ml Tropftrichter überführt und unter Rühren bei -70°C in eine Lösung von Borsäuretrimethylester (8.9 g; 9.6 ml; 86 mmol) in 50 ml trockenem THF getropft, wobei sich ein Niederschlag bildete. Man ließ über Nacht auf RT erwärmen und trug die Reaktionsmischung unter Rühren in eine Mischung aus 100 g Eis und 3 ml konz. Schwefelsäure ein. Die organische Phase wurde abgetrennt und die wäßrige Phase 3x mit je 100 ml Chloroform extrahiert, die vereinigten organischen Phasen wurden eingeengt. Das Rohprodukt wurde anschließend aus Hexan umkristallisiert.
   Produkt: farbloser, wachsartiger Feststoff (11.28 g; 66 %); Schmelzpunkt: 84-87°C.
   ¹H NMR (400 MHz; CDCl₃): δ [ppm] = 8.15, 7.00 (AA'BB'; 4 H; H-aryl), 4.07 (t; 2 H; J = 7.7 Hz; O-CH₂), 1.83 (pseudo-quin; 2 H; J = 7.5 Hz; O-CH₂-CH₂), 1.55-1.32 (m; 6 H; H-alkyl), 0.93 (pseudo-t; 3 H; J = 7.7 Hz; CH₃). Enthielt variable Anteile an Anhydriden.

### Beispiel B2: Synthese von 3-(3,7-Dimethyloctyloxy)benzolboronsäure:

a) Synthese von 3-(3,7-Dimethyloctyloxy)brombenzol:
   450 ml Ethanol wurden vorgelegt und mit Nal (10.5 g; 70 mmol) und KOH (67.3 g; 1,2 mol) versetzt. Man beobachtete einen Temperaturanstieg nach KOH-Zugabe von 25 auf 40 °C. Nach Abkühlen auf Raumtemperatur wurde 3-Bromphenol (176.5 g; 1 mol) zugegeben. Die weiße Suspension wurde dabei beige. 3,7-Dimethyloctylchlorid (186.32 g; 212.94 ml; 1,05 mol) wurde über einen Tropftrichter innerhalb 3 min. zugegeben. Es wurde 2 Stunden bei RT nachgerührt und anschließend für 96 Stunden bei 80°C Innentemperatur gerührt. Ethanol wurde abdestilliert. Der Rückstand wurde in Ethylacetat aufgenommen und der Niederschlag wurde durch Filtration separiert. Die organische Phase wurde mit 10 gew.-%iger wäßriger NaOH-Lösung dreimal extrahiert, einmal mit H₂O gewaschen, dreimal mit H₂O, das mit CO₂ angesäuert wurde, und nochmals mit H₂O gewaschen. Nach Trocknung mit MgSO₄ wurde erneut am Rotationsverdampfer das Lösungsmittel abgezogen und das Rohprodukt durch fraktionierte Vakuumdestillation gereinigt.
   Produkt: hochsiedendes farbloses Öl; 180°C bei 2-3 mbar; 262,3 g (84%)
   ¹H NMR (400 MHz; CDCl₃): δ [ppm] = 7.12 (pseudo-t; 1 H; J = 8 Hz; H-5), 7.05 (m; 2 H; H-2, H-6), 6.81 (ddd; 1 H; J₁ = 8, J₂ = 2, J₃ = 0.7 Hz; H-4), 3.97 (m; 2 H; O-CH₂), 1.81 (m; 1 H; O-CH₂-CH₂-CH), 1.70-1.50 (m; 3 H; H-alkyl), 1.35-1.13 (m; 6 H; H-alkyl), 0.93 (d; 3 H; J = 7.7 Hz; CH₃), 0.87 (d; 6 H; J = 7.7 Hz; CH₃).
b) Synthese von 3-(3,7-Dimethyloctyloxy)benzolboronsäure:
   Mg-Späne (24.7 g, 1.02 mol) wurden vorgelegt und die Apparatur unter Argon ausgeheizt. Bei Raumtemperatur wurden ca. 100 ml THF über den Tropftricher eingefüllt, sowie einige Kristalle lod zugegeben. Anschließend tropfte man in die ruhende Lösung einige ml 3-(3,7-Dimethyloctyloxy)-brombenzol und erhitzte mit einem Heißluftgebläse an der Eintropfstelle. Nachdem die Reaktion gestartet war, ließ man unter Rühren das restliche 3-(3,7-Dimethyloctyloxy)-brombenzol (gesamt: 313 g, 1 mol, 280 ml) kontinuierlich zutropfen (70 min). Gleichzeitig wurden weitere 1100 ml THF zugegeben. Der Reaktionsansatz wurde für weitere zwei Stunden am Rückfluß gerührt.
   Das erhaltene, auf Raumtemperatur abgekühlte Grignard-Reagenz wurde unter Schutzgas und schnellem Rühren so zu einer auf -70°C gekühlten Mischung aus 800 ml THF sowie 123 ml Trimethylborat (114 g, 1.10 mol) getropft, daß die Innentemperatur -60°C nicht überschritt (Dauer: 3 h). Es bildete sich eine helle Suspension.
   Die Reakionsmischung wurde in 1200 g Eiswasser/40 ml konz. H₂SO₄ eingerührt. Die klaren Phasen wurden getrennt und die Wasserphase mit Ethylacetat ausgeschüttelt. Die vereinigten organischen Phasen wurden mit Wasser gerührt und nach Trocknen eingeengt.
   Zur weiteren Reinigung wurde der so erhaltene farblose Feststoff mit ca. 500 ml Hexan (welches mit 2 ml konz. wäßriger HCI versetzt wurden) gerührt.
   Man erhielt 239 g (86%) farbloses Kristallpulver.
   Schmelzpunkt: 83-89°C.
   ¹H NMR (400 MHz; CDCl₃): δ [ppm] = 7.81 (td; 1 H; J₁ = 8, J₂ = 1.3 Hz; H-4), 7.73 (dd; 1 H; J₁ = 2, J₂ = 1.1 Hz; H-2), 7.43 (t; 1 H; J = 8 Hz; H-5), 7.13 (ddd; 1 H; J₁ = 8, J₂ = 2, J₃ = 1.1 Hz; H-6), 4.11 (m; 2 H; O-CH₂), 1.90 (m; 1 H; O-CH₂-CH₂-C*H*), 1.75-1.50 (m; 3 H; H-alkyl), 1.44-1.14 (m; 6 H; H-alkyl), 1.00 (d; 3 H; J = 7.9 Hz; CH₃), 0.88 (d; 6 H; J = 7.8 Hz; CH₃). Enthält variable Anteile an Anhydriden.

### Beispiel B3: Synthese von 2,5-Dimethylbenzolboronsäure:

In eine ausgeheizte, Argon geflutete Apparatur werden Magnesiumspäne (13,3 g; 0,55 mol) eingefüllt, mit ca. 30 ml THF überlagert und einigen Kristallen lod versetzt. Anschließend tropfte man in die ruhende Lösung einige Tropfen Brom-p-xylol (Vgl. Beispiel **A1** a)) zu. Die Grignardreaktion begann sehr schnell, anschließend tropfte man unter Rühren das restliche Brom-p-xylol (Gesamtmenge: 92.5 g; ca. 70 ml; 0.5 mol) weiter zu. Es wurde für 4 Stunden am Rückfluß gekocht, danach abgekühlt. Die Grignardlösung wurde dann im Schutzgasgegenstrom in einen 500 ml Tropftrichter überführt und unter Rühren bei -70°C in eine Lösung von Borsäuretrimethylester (62.4 g; 67 ml; 0.6 mol) in 350 ml THF getropft (Dauer ca. 1 h). Dabei fiel ein Niederschlag aus. Man ließ über Nacht auf RT erwärmen und trug die Reaktionsmischung unter Rühren in eine Mischung aus 700 g Eis und 20 ml konz. Schwefelsäure ein. Die organische Phase wurde abgetrennt, die wäßrige Phase dreimal mit Chloroform extrahiert und die vereinigten organischen Phasen eingeengt. Das Rohprodukt wurde aus Chloroform/Hexan umkristallisiert.
Man erhielt ein farbloses mikrokristallines Pulver: 47.71 g (64 %).
¹H NMR (400 MHz; CDCl₃): δ [ppm] = 8.00 (d; 1 H; J = 1.4 Hz; H-6), 7.26 (dd; 1 H; J₁ = 8.0, J₂ = 1.4 Hz; H-4), 7.17 (d; 1 H; J = 8 Hz; H-3), 2.76, 2.38 (jeweils: s; 3 H; CH₃). Enthielt variable Anteile an Anhydriden.

### Beispiel B4: Synthese von 4-(3,7-Dimethyloctyloxy)benzolboronsäure:

a) Synthese von 4-(3,7-Dimethyloctyloxy)-brombenzol
   Durchführung analog zu Beispiel B2, a).
   Ausbeute: 85%
   Siedepunkt: 180°C bei 2 mbar
   ¹H NMR (CDCl₃): δ [ppm] = 7.36, 6.77 (AA'BB', 4 H, H-Aryl), 3.95 (m, 2 H, O-CH₂), 1.82 (m, 1 H, H-3'), 1.6 (m, 3 H, H-2', H-7'), 1.24 (m, 6 H, H-4', H-5', H-6'), 0.94 (d, 3 H, Me, J = 7 Hz), 0.87 (d, 6 H, Me, J = 7 Hz).
b) Synthese von 4-(3,7-Dimethyloctyloxy)benzolboronsäure
   Durchführung analog zu Beispiel B2, b).
   Ausbeute: 83%
   Schmelzpunkt: 57-63°C.
   ¹H NMR (CDCl₃): δ [ppm] = 7.67, 6.92 (AA'BB', 4 H, H-Aryl), 4.6 (br, 2 H, B(OH)₂), 4.03 (m, 2 H, O-CH₂), 1.87 (m, 1 H, H-3'), 1.65 (m, 3 H, H-2', H-7'), 1.27 (m, 6 H, H-4', H-5', H-6'), 0.95 (d, 3 H, Me, J = 7 Hz), 0.87 (d, 6 H, Me, J = 7 Hz). Enthält variable Anteile von Anhydriden.

### Beispiel B5: Synthese von 3,4-Bis(-2-methylpropyloxy)benzolboronsäure

a) Synthese von 1,2-Bis(-2-methylpropyloxy)benzol:
   Brenzkatechin (220.22 g, 2 mol) und Nal (10.49 g, 0.14 mol) wurden in 900 ml Ethanol vorgelegt und zum Rückfluß erhitzt. Anschließend wurden in ca. 300 ml Ethanol gelöstes KOH (56.11 g, 1 mol) und gleichzeitig 1-Brom-2-methylpropan (137.03 g, 1 mol, 108.75 ml) langsam zugetropft. Es wurde über Nacht weiter am Rückfluß gekocht. Am nächsten Tag wurden erneut dieselbe Menge KOH und Alkylbromid zugegeben. Insgesamt wurde dieser Vorgang siebenmal wiederholt. Nach Abkühlen der Reaktionsmischung wurde vom Feststoff abdekantiert. Der Filterkuchen wurde mit Ethanol nachgewaschen. Die organische Phase wurde eingeengt. Der Filterkuchen wurde in 11 warmen Wasser aufgelöst und mit der mit Ethylacetat verdünnten organischen Phase versetzt. Nach Phasentrennung wurde wiederholt mit 10 %iger wäßriger NaOH gerührt, mit Wasser ausgewaschen und über Na₂SO₄ getrocknet. Das nach Abziehen des Lösungsmittels erhaltene Rohprodukt wurde im Vakuum fraktioniert destilliert.
   Man erhielt das Produkt als farbloses Öl (Siedepunkt: 82°C bei 0.18 mbar): 333.4 g (75%).
   ¹H NMR (CDCl₃): δ [ppm] = 6.87 (ps-s, 4 H, H-Aryl), 3.75 (d, 4 H, O-CH₂, J = 8 Hz), 2.13 (ps-non, 2 H, C-H, J = 8 Hz), 1.05 (d, 12 H, CH₃, J = 8 Hz).
b) Synthese von 3,4-Bis(2-methylpropyloxy)brombenzol:
   1,2-Bis(-2-methylpropyloxy)benzol (359.61 g, 1.62 mol) wurde mit 500 ml CH₂Cl₂ vorgelegt und mit wenig Eisenpulver versetzt. Nun tropfte man unter Kühlung langsam Brom (266.88 g, 1.78 mol) (vermischt mit ca. 200 ml CH₂Cl₂) zu. Der Ansatz wurde für ca. 20 h bei Raumtemperatur gerührt. Zur Aufarbeitung wurde der Ansatz mit wäßriger Na₂SO₃ Lösung ausgerührt und anschließend das Eisenpulver abfiltriert. Die organische Phase wurde dann noch 2x mit NaHCO₃ Lösung ausgeschüttelt und anschließend mit Wasser neutral gewaschen. Nach Trocknung wurde die organische Phase eingeengt.
   Durch doppelte fraktionierte Destillation erhielt man das gewünschte Produkt als farblosen Feststoff (166.9 g, 34%).
   Schmelzpunkt: 47°C
   ¹H NMR (CDCl₃): δ [ppm] = 6.98 (m, 2 H, H-2, H-6), 6.73 (m, 1 H, H-5), 3.72, 3.70 (2 x d, 2 x 2 H, O-CH₂, J = 8 Hz), 2.12 (m, 2 H, CH), 1.04 (m, 12 H, CH₃).
c) Synthese von 3,4-Bis(2-methylpropyloxy)benzolboronsäure:
   Durchführung analog zu Beispiel B2, b).
   Ausbeute: 76%
   Schmelzpunkt: 146°C.
   ¹H NMR (CDCl₃): δ [ppm] = 7.81 (dd, 1 H, H-6, J₁ = 8 Hz, J₂ = 1.8 Hz), 7.68 (d, 1 H, H-2, J = 1.8 Hz), 6.99 (d, 1 H, H-5, J = 8 Hz), 3.89, 3.84 (2 x d, 2 x 2 H, O-CH₂, J = 8 Hz), 2.13 (m, 2 H, CH), 1.07 (m, 12 H, CH₃). Enthält variable Anteile von Anhydriden.

### Beispiel B6: Synthese von 4'-(3,7-Dimethyloctyloxy)-biphenyl-4-boronsäure

a) Synthese von 4-(3,7-Dimethyloctyloxy)-4'-brombiphenyl:
   Durchführung analog zu Beispiel B2, a).
   Aufarbeitung durch Umkristallisation aus Ethanol.
   Farblose Kristalle, 85% Ausbeute.
   Schmelzpunkt: 104°C
   ¹H NMR (CDCl₃): δ [ppm] = 7.53, 7.40 (AA'BB', 4 H, H-Aryl), 7.47, 6.96 (AA'BB', 4 H, H-Aryl), 4.03 (m, 2 H, O-CH₂), 1.83 (m, 1 H, H-3'), 1.62 (m, 3 H, H-2', H-7'), 1.3 (m, 6 H, H-4', H-5', H-6'), 0.96 (d, 3 H, Me, J = 7.5 Hz), 0.87 (d, 6 H, Me, J = 7.5 Hz).
b) Synthese von 4'-(3,7-Dimethyloctyloxy)-biphenyl-4-boronsäure:
   Durchführung analog zu Beispiel B2, b).
   Ausbeute: 78%
   Schmelzpunkt: 116°C
   ¹H NMR (DMSO): δ [ppm] = 8.02 (br, 2 H, B(OH)₂), 7.83, 7.58 (AA'BB', 4 H, H-Aryl), 7.61, 7.01 (AA'BB', 4 H, H-Aryl), 4.04 (m, 2 H, O-CH₂), 1.77 (m, 1 H, H-3'), 1.58 (m, 3 H, H-2', H-7'), 1.25 (m, 6 H, H-4', H-5', H-6'), 0.92 (d, 3 H, Me, J = 7.5 Hz), 0.86 (d, 6 H, Me, J = 7.5 Hz).

### C. Kupplungsreaktionen gemäß Reaktion A

### Beispiel C1: Synthese von 2-(4'-Hexyloxyphenyl)terephthalsäurediethylester:

Bromterephthalsäurediethylester (30.1 g, 100 mmol), K₂CO₃ (27.6 g, 200 mmol) und 140 ml Toluol und 140 ml H₂O wurden vorgelegt und 30 Minuten mit Argon gespült.

Anschließend wurde 4-Hexyloxyphenylboronsäure (26.7 g, 120 mmol) (vgl. **B1**), sowie Pd(PPh₃)₄ (1.16 g, 1 mmol) unter Schutzgas zugegeben. Das gelb-grünliche, trübe Gemisch wurde unter Schutzgasüberlagerung bei 85° C Innentemperatur kräftig gerührt. Nach 7 Stunden war die Reaktion beendet. Nach Phasentrennung wurde die organische Phase mit verdünnter HCI / H₂O ausgeschüttelt (neutral). Die wäßrige Phase schüttelte man mit Toluol aus und vereinigt die organischen Phasen. Nach Abfiltrieren von eventuellen Palladium-Resten wurde eingeengt. Man erhielt das Produkt als gelb-braunes Öl in ausreichender Reinheit (ca. 85%): 44.7 g (112%).
¹H NMR (400 MHz; CDCl₃): δ [ppm] = 8.03 (dd; 1 H; J₁ = 2, J₂ = 1 Hz; H-3), 8.02 (dd; 1 H; J₁ = 8, J₂ = 2 Hz; H-5), 7.79 (dd; 1 H; J₁ = 8, J₂ = 1 Hz; H-6), 7.25, 6.93 (AA'BB'; 4 H; H-phenyl), 4.40, 4.14 (jeweils: q; 2 H; J = 8 Hz; CO₂-CH₂), 3.99 (t; 2 H; J = 7.5 Hz; O-CH₂), 1.81 (m; 2 H; O-CH₂-CH₂), 1.53-1.33 (m; 6 H; H-alkyl), 1.40, 1.07 (jeweils: t; 3 H; J = 8 Hz; CO₂-CH₂-CH₃ ), 0.91 (m; 3 H; CH₃).

### Beispiel C2: Synthese von 2-(3'-(3,7-Dimethyloctyloxy)phenyl)terephthalsäuredimethylester:

Bromterephthalsäuredimethylester (49.7 g, 182 mmol, von TransWorld, Rockville MD, USA, bezogen bzw. analog Beispiel A1 c) hergestellt), K₂CO₃ (50.3 g, 364 mmol) und 170 ml Toluol und 170 ml H₂O wurden vorgelegt und 30 Minuten mit Argon gespült. Anschließend wurde 3-(3,7-Dimethyloctyloxy )boronsäure (55.7 g, 200 mmol) (vgl. B2), sowie Pd(PPh₃)₄ (0.93 g, 0.8 mmol) unter Schutzgas zugegeben. Das gelb-grünliche, trübe Gemisch wurde unter Schutzgasüberlagerung bei 85° C Innentemperatur kräftig gerührt. Nach 24 Stunden war die Reaktion beendet. Nach Phasentrennung wurde die organische Phase mit verdünnter HCI / H₂O ausgeschüttelt (neutral). Die wäßrige Phase schüttelte man mit Ethylacetat aus und vereinigte die organischen Phasen. Diese wurden eingeengt und bei 2 mbar getrocknet. Man erhielt das Produkt als gelbes Öl in ausreichender Reinheit (größer 95%): 76.1 g (98%).
¹H NMR (400 MHz; CDCl₃): δ [ppm] = 8.07 (d; 1 H; J = 2 Hz; H-3), 8.05 (dd; 1 H; J₁ = 8, J₂ = 2 Hz; H-5), 7.82 (d; 1 H; J = 8 Hz; H-6), 7.29 (t; 1 H; J = 8 Hz; H-5'), 6.90 (m; 3 H; H-2', H-4', H-6'), 4.01 (m; 2 H; O-CH₂), 3.94, 3.67 (jeweils: s; 3 H; CO₂-CH₃), 1.84 (m; 1 H; O-CH₂-CH₂-CH), 1.63-1.48 (m; 3 H; H-alkyl), 1.37-1.12 (m; 6 H; H-alkyl), 0.96 (d; 3 H; J = 7.8 Hz; CH₃), 0.87 (d; 6 H; J = 7.7 Hz; CH₃).

### Beispiel C3: Synthese von 2-(2',5'-Dimethylphenyl)terephthalsäurediethylester:

Bromterephthalsäurediethylester (45.2 g, 150 mmol), K₂CO₃ (41.5 g, 300 mmol), 140 ml Toluol und 140 ml H₂O wurden vorgelegt und 30 Minuten mit Argon gespült. Anschließend wurde 2,5-Dimethylbenzolboronsäure (24.8 g, 165 mmol) (vgl. **B3**), sowie Pd(PPh₃)₄ (0.7 g, 0,6 mmol) unter Schutzgas zugegeben. Das bräunliche, durch Phasentrennung trübe Gemisch wurde unter Schutzgasüberlagerung bei 85° C Innentemperatur kräftig gerührt. Die Reaktion war nach 24 Stunden (laut DC) beendet. Nach Phasentrennung wurde die org. Phase mit verdünnter HCI / H₂O ausgeschüttelt (neutral). Die wäßrige Phase schüttelte man mit Toluol aus und vereinigte die organischen Phasen. Nach Abfiltrieren von evtl. Palladium-Resten wurde eingeengt. Man erhielt das Produkt als gelbes Öl in ausreichender Reinheit (größer 97%). Ausbeute: 48.7 g (99 %).
¹H NMR (400 MHz; CDCl₃): δ [ppm] = 8.07 (dd; 1 H; J₁ = 8, J₂ = 2 Hz; H-5), 7.96 (d; 1 H; J = 8 Hz; H-6), 7.92 (d; 1 H; J = 2 Hz; H-3), 7.14 (d; 1 H; J = 7.9 Hz; H-3'), 7.09 (dd; 1 H; J₁ = 7.9, J₂ = 2 Hz; H-4'), 6.91 (d; 1 H; J = 2 Hz; H-6'), 4.39, 4.16 (jeweils: q; 2 H; J = 8 Hz; CO₂-CH₂), 2.32, 2.02 (jeweils: s; 3 H; aryl-CH₃), 1.39, 0.97 (jeweils: t; 3 H; J = 8 Hz; CO₂-CH₂-CH₃).

### Beispiel C4: Synthese von 4'-(3",7"-Dimethyloctyloxyphenyl)terephthalsäurediethylester

Durchführung analog Beispiel C3; Palladiumreste wurden noch durch Ausrühren mit mit 1 %iger wäßriger NaCN-Lösung beseitigt.
Das Produkt (100% Ausbeute) ist ein farbloses hochviskoses Öl.
¹H NMR (CDCl₃): δ [ppm] = 8.04 (d, 1 H, H-3, J = 1.8 Hz), 8.03 (dd, 1 H, H-5, J₁ = 7.8, J₂ = 1.8 Hz), 7.8 (d, 1 H, H-6, J = 7.8 Hz), 7.25, 6.93 (AA'BB', 4 H, H-Aryl), 4.40, 4.15 (2 x q, 2 x 2 H, CO₂CH₂, J = 7.6 Hz), 4.04 (m, 2 H, O-CH₂), 1.86 (m, 1 H, H-3"), 1.60 (m, 3 H, H-2", H-7"), 1.40, 1.07 (2 x t, 2 x 3H, Ester-CH₃, J = 7.6 Hz), 1.30 (m, 6 H, H-4", H-5", H-6"), 0.92 (d, 3 H, Me, J = 7.5 Hz), 0.86 (d, 6 H, Me, J = 7.5 Hz).

### Beispiel C5: Synthese von 3,4-Bis(2-methylpropyloxy)phenylterephthalsäurediethylester

Synthese analog Beispiel C4. Das Produkt (99% Ausbeute) ist ein farbloses hochviskoses Öl.
¹H NMR (CDCl₃): δ [ppm] = 8.05 (d, 1 H, H-3, J = 1.9 Hz), 8.03 (dd, 1 H, H-5, J₁ = 7.9, J₂ = 1.9 Hz), 7.77 (d, 1 H, H-6, J = 7.9 Hz), 6.87 (m, 3 H, H-Aryl), 4.40, 4.13 (2 x q, 2 x 2 H, CO₂CH₂, J = 7.5 Hz), 3.79, 3.76 (2 x d, 2 x 2 H, O-CH₂, J = 8 Hz), 2.13 (m, 2 H, CH), 1.41, 1.07 (2 x t, 2 x 3H, Ester-CH₃, J = 7.5 Hz), 1.04 (m, 12 H, CH₃).

### Beispiel C6: Synthese von 4-[4'-(3,7-Dimethyloctyloxy)biphenyl]terephthalsäurediethylester

Synthese analog Beispiel C4. Das Produkt (99% Ausbeute) ist ein farbloses hochviskoses Öl.
¹H NMR (CDCl₃): δ [ppm] = 8.10 (d, 1 H, H-3, J = 1.9 Hz), 8.07 (dd, 1 H, H-5, J₁ = 7.9, J₂ = 1.9 Hz), 7.86 (d, 1 H, H-6, J = 7.9 Hz), 7.59, 7.38 (AA'BB', 4 H, H-Aryl), 7.56, 6.99 (AA'BB', 4 H, H-Aryl), 4.41, 4.14 (2 x q, 2 x 2 H, CO₂CH₂, J = 7.6 Hz), 4.05 (m, 2 H, O-CH₂), 1.86 (m, 1 H, H-3"), 1.65 (m, 3 H, H-2", H-7"), 1.41, 1.04 (2 x t, 2 x 3H, Ester-CH₃, J = 7.6 Hz), 1.30 (m, 6 H, H-4", H-5", H-6"), 0.96 (d, 3 H, Me, J = 7.5 Hz), 0.87 (d, 6 H, Me, J = 7.5 Hz).

### Beispiel C7: Synthese von 2-[4-(3,7-Dimethyloctyloxy)phenyl]-5-methoxyterephthalsäurediethylester

Synthese analog Beispiel C4 (hier unter Verwendung von 2-Brom-5-methoxyterephthalsäurediethylester, vgl. Bsp. A2). Das Produkt (95% Ausbeute) war ein farbloses hochviskoses Öl.
¹H NMR (CDCl₃): δ [ppm] = 7.75, 7.35 (2 x s, 2 x 1 H, H-3, H-6), 7.20, 6.91 (AA'BB', 4 H, H-Aryl), 4.37, 4.12 (2 x q, 2 x 2 H, CO₂CH₂, J = 7.6 Hz), 4.02 (m, 2 H, O-CH₂), 3.97 (s, 3 H, O-Me), 1.84 (m, 1 H, H-3"), 1.62 (m, 3 H, H-2", H-7"), 1.37, 1.03 (2 x t, 2 x 3H, Ester-CH₃, J = 7.6 Hz), 1.28 (m, 6 H, H-4", H-5", H-6"), 0.96 (d, 3 H, Me, J = 7.5 Hz), 0.87 (d, 6 H, Me, J = 7.5 Hz).

### Beispiel C8: Synthese von 2-[3-(3,7-Dimethyloctyloxy)phenyl]-5-methoxyterephthalsäurediethylester

Synthese analog Beispiel C7. Das Produkt (95% Ausbeute) war ein farbloses hochviskoses Öl.
¹H NMR (CDCl₃): δ [ppm] = 7.78, 7.37 (2 x s, 2 x 1 H, H-3, H-6), 7.26 (t; 1 H; H-5', J = 8 Hz), 6.86 (m; 3 H; H-2', H-4', H-6'), 4.37, 4.10 (2 x q, 2 x 2 H, CO₂CH₂, J = 7.6 Hz), 4.00 (m, 2 H, O-CH₂), 3.97 (s, 3 H, O-Me), 1.83 (m, 1 H, H-3"), 1.62 (m, 3 H, H-2", H-7"), 1.37, 1.01 (2 x t, 2 x 3H, Ester-CH₃, J = 7.6 Hz), 1.28 (m, 6 H, H-4", H-5", H-6"), 0.95 (d, 3 H, Me, J = 7.5 Hz), 0.86 (d, 6 H, Me, J = 7.5 Hz).

### D. Reduktionen gemäß Reaktion B

### Beispiel D1: Synthese von 2,5-Bishydroxymethyl-4'-hexyloxybiphenyl:

LiAlH₄ (5,3 g, 140 mmol) wurde in ca. 200 ml THF mit Argonüberlagerung vorgelegt und 2-(4'-Hexyloxyphenyl)terephthalsäurediethylester (40 g, 100 mmol) (vgl. C1) mit weiteren 50 ml THF über einen Tropftrichter langsam zugetropft. Dabei wurde die Reaktionsmischung kräftig gerührt. Anschließend kocht man für ca. eine Stunde am Rückfluß. Die Reaktionsmischung wurde auf RT gebracht und unter Wasserbadkühlung sowie Argonüberlagerung vorsichtig bis zum Ende der Gasentwicklung tropfenweise mit Eiswasser versetzt. Anschließend wurde solange verdünnte (10 %-ig) Schwefelsäure zugetropft, bis die trüb-graue Mischung klar war. Die Phasen wurden durch Chloroformzugabe getrennt und die wäßrige Phase mit Chloroform (zweimal) ausgeschüttelt. Die organischen Phasen wurden einmal mit H₂O gewaschen und anschließend eingeengt. Das erhaltene Rohprodukt wurde aus Hexan/Ethylacetat (5/1) umkristallisiert.
Produkt: 20,3 g (65%) farblose Nadel, Reinheit > 98%. Schmelzpunkt: 72.5-74°C.
¹H NMR (400 MHz; CDCl₃): δ [ppm] = 7.53 (d; 1 H; J = 8 Hz; H-6), 7.36 (dd; 1 H; J₁ = 8, J₂ = 2 Hz; H-5), 7.27 (d; 1 H; J = 2 Hz; H-3), 7.26, 6.94 (AA'BB'; 4 H; H-phenyl), 4.72, 4.61 (jeweils: s; 2 H; CH₂-O), 3.99 (t; 2 H; J = 7.5 Hz; O-CH₂), 1.81 (m; 2 H; O-CH₂-CH₂), 1.53-1.26 (m; 6 H; H-alkyl), 0.92 (m; 3 H; CH₃).

### Beispiel D2: Synthese von 2,5-Bishydroxymethyl-3'-(3,7-dimethyloctyloxy)biphenyl:

LiAlH₄ (9.4 g, 248 mmol) wurde unter N₂ in 300 ml THF vorgelegt. Bei RT wurde dann langsam 2-(3'-(3,7-Dimethyloctyloxy)phenyl)terephthalsäuredimethylester (75.5 g, 177 mmol), gelöst in 120 ml THF, zutropft. Anschließend wurde 4 h unter Rückfluß gerührt. Nach dem Abkühlen wurde überschüssiges LiAlH₄ vorsichtig durch H₂O-Zugabe vernichtet. Anschließend wurde halbkonzentrierte H₂SO₄ vorsichtig ( ca. 50 ml ) zugetropft. Der Ansatz war dabei sehr zähflüssig. Nach 1 h Nachrührzeit war eine klare Lösung und unten im Kolben ein schleimiger grauer Niederschlag zu sehen. Die klare Lösung wurde abdekantiert und das Lösungsmittel abgezogen. Der zurückgebliebene Niederschlag wurde mit viel Wasser und Ethylacetat gerührt, nach Filtration die organische Phase abgetrennt, das Lösungsmittel abgezogen und mit der ersten organischen Phase vereinigt. Die vereinigten organischen Phasen wurden in Ethylacetat aufgenommen und mit Wasser fünfmal extrahiert. Nach Trocknung über MgSO₄ wurde das Lösungsmittel abgezogen. Das entstehende Öl wurde mehrfach mit Hexan gerührt und am Ölpumpenvakuum getrocknet. Das Produkt wurde so als reines hellgelbes, hochviskoses Öl (54 g, 82 %) erhalten.
¹H NMR (400 MHz; CDCl₃): δ [ppm] = 7.50 (d; 1 H; J = 7.8 Hz; H-6), 7.34 (dd; 1 H; J₁ = 7.8, J₂ = 1.9 Hz; H-5), 7.30 (dt; 1 H; J₁ = 8, J₂ = 1 Hz; H-5'), 7.26 (d; 1 H; J = 1.9 Hz; H-3), 6.88 (m; 3 H; H-2', H-4', H-6'), 4.69, 4.59 (jeweils: s; 2 H; CH₂-OH), 4.00 (m; 2 H; O-CH₂), 1.97 (s; 2 H; OH), 1.82 (m; 1 H; O-CH₂-CH₂-CH), 1.67-1.50 (m; 3 H; H-alkyl), 1.40-1.13 (m; 6 H; H-alkyl), 0.95 (d; 3 H; J = 7.5 Hz; CH₃), 0.87 (d; 6 H; J = 7.6 Hz; CH₃).

### Beispiel D3: Synthese von 2,5-Bishydroxymethyl-2',5'-dimethylbiphenyl:

LiAlH₄ (7.9 g, 208 mmol) wurde unter Argonüberlagerung mit ca. 250 ml THF vorgelegt. 2-(2',5'-Dimethylphenyl)terephthalsäurediethylester (48.6 g, 149 mmol) (vgl. C3) wurde im Tropftrichter mit etwa 60 ml THF verdünnt und langsam zugetropft. Dabei wurde die Reaktionsmischung kräftig gerührt. Der mit nochmals 100 ml THF verdünnte Ansatz wurde dann bei 67°C am Rückfluß gekocht. Nach 2 h wurde auf RT abgekühlt. Unter Wasserbadkühlung und Argonüberlagerung wurde bis zum Ende der Gasentwicklung tropfenweise mit Eiswasser versetzt. Anschließend wurde solange verdünnte (10 %-ige) Schwefelsäure zugetropft, bis die trüb-graue Mischung aufklarte. Das Phasengemisch wurde durch großzügige Chloroformzugabe getrennt und anschließend die wäßrige Phase zweimal mit Chloroform ausgeschüttelt. Die organischen Phasen wurden einmal mit H₂O geschüttelt und eingeengt. Das Rohprodukt wurde aus Chloroform/Hexan umkristallisiert: 24.7 g (68 %) farbloses, mikrokristallines Pulver; Schmelzpunkt: 145 - 148° C (Reinheit > 95%).
¹H NMR (400 MHz; CDCl₃): δ [ppm] = 7.54 (d; 1 H; J = 7.8 Hz; H-6), 7.38 (dd; 1 H; J₁ = 7.8, J₂ = 1.8 Hz; H-5), 7.15 (d; 1 H; J = 7.8 Hz; H-3'), 7.13 (d; 1 H; J = 1.9 Hz; H-3), 7.08 (dd; 1 H; J₁ = 7.7, J₂ = 1.5 Hz; H-4'), 6.94 (d; 1 H; J = 1.5 Hz; H-6'), 4.72, 4.42 (jeweiis: s; 2 H; CH₂-O), 2.33, 2.01 (jeweils: s; 3 H; aryl-CH₃).

### Beispiel D4: Synthese von 2,5-Bishydroxymethyl-4'-(3,7-dimethyloctyloxy)biphenyl

Durchführung analog Beispiel D3; der Ansatz wurde jedoch nicht sauer, sondern alkalisch aufgearbeitet: dazu gab man nach beendeter Reduktion vorsichtig x ml Wasser (bei einem Einsatz von x g LiAlH₄) zu. Anschließend wurden x ml wäßriger NaOH-Lösung (15%ig) und schließlich 3 x ml Wasser zugegeben. Nach jeder Zugabe wurde für ca. 15 Minuten nachgerührt ("1:1:3 Methode"). Von dem entstandenen Feststoff wurde abgesaugt, dieser erneut mit THF gerührt und schließlich die vereinigten organischen Phasen eingeengt. Diese Aufarbeitung erwies sich als vorteilhafter im Vergleich zu der saueren Variante, die in den Beispielen D1 bis D3 Verwendung fand. Umkristallisation aus Hexan/Ethylacetat (30:1).
Man erhielt das Produkt (88% Ausbeute) als farblosen, wachsartigen Feststoff. Schmelzpunkt: 67°C
¹H NMR (CDCl₃): δ [ppm] = 7.53 (d, 1 H, H-6, J = 7.9 Hz), 7.36 (dd, 1 H, H-5, J₁ = 7.9, J₂ = 2 Hz), 7.27 (d, 1 H, H-3, J = 2 Hz), 7.28, 6.95 (AA'BB', 4 H, H-Aryl), 4.72, 4.63 (2 x d, 2 x 2 H, CH₂O, J = 8 Hz), 4.03 (m, 2 H, O-CH₂), 1.90, 1.68 (2 x t, 2 x 1 H, OH, J = 8 Hz), 1.85 (m, 1 H, H-3'), 1.65 (m, 3 H, H-2', H-7'), 1.30 (m, 6 H, H-4', H-5', H-6'), 0.97 (d, 3 H, Me, J = 7.5 Hz), 0.87 (d, 6 H, Me, J = 7.5 Hz).

### Beispiel D5: Synthese von 2,5-Bishydroxymethyl-3',4'-bis(2-methylpropyloxy)biphenyl

Synthese analog Beispiel D4. Umkristallisation aus Hexan/Ethylacetat (15:1). Man erhielt das Produkt (84% Ausbeute) als farblose Kristalle.
Schmelzpunkt: 73°C
¹H NMR (CDCl₃): δ [ppm] = 7.53 (d, 1 H, H-6, J = 7.9 Hz), 7.37 (dd, 1 H, H-5, J₁ = 7.9, J₂ = 2 Hz), 7.29 (d, 1 H, H-3, J = 2 Hz), 6.89 (m, 3 H, H-Aryl), 4.73, 4.63 (2 x s, 2 x 2 H, CH₂O), 3.80, 3.77 (2 x d, 2 x 2 H, O-CH₂, J = 8 Hz), 2.15 (m, 2 H, CH), 1.55 (br, 2 H + H₂O, OH), 1.06, 1.03 (2 x t, 2 x 6 H, CH₃).

### Beispiel D6: Synthese von 2,5-Bishydroxymethyl-4"-(3,7-dimethyloctyloxy)terphenyl

Synthese analog Beispiel D4. Umkristallisation aus Hexan/Ethylacetat (15:1). Man erhielt das Produkt (88% Ausbeute) als farblose Kristalle.
Schmelzpunkt: 106°C
¹H NMR (CDCl₃): δ [ppm] = 7.60, 7.41 (AA'BB', 4 H, H-Aryl), 7.56, 6.99 (AA'BB', 4 H, H-Aryl), 7.54 (d, 1 H, H-6, J = 7.9 Hz), 7.39 (dd, 1 H, H-5, J₁ = 7.9, J₂ = 2 Hz), 7.32 (d, 1 H, H-3, J = 2 Hz), 4.74, 4.66 (2 x d, 2 x 2 H, CH₂O, J = 4 Hz), 4.05 (m, 2 H, O-CH₂), 1.87 (m, 1 H, H-3'), 1.77, 1.67 (2 x br, 2 x 1 H, OH), 1.65 (m, 3 H, H-2', H-7'), 1.27 (m, 6 H, H-4', H-5', H-6'), 0.96 (d, 3 H, Me, J = 7.5 Hz), 0.88 (d, 6 H, Me, J = 7.5 Hz).

### Beispiel D7: Synthese von 2,5-Bishydroxymethyl-4-methoxy-4'-(3,7-dimethyloctyloxy)biphenyl

Synthese analog Beispiel D4. Umkristallisation aus Hexan/Ethylacetat (20:1). Man erhielt das Produkt (93% Ausbeute) als farblose Kristalle.
Schmelzpunkt: 101°C
¹H NMR (CDCl₃): δ [ppm] = 7.21, 6.93 (AA'BB', 4 H, H-Aryl), 7.18, 7.10 (2 x s, 2 x 1 H, H-3, H-6), 4.70, 4.62 (2 x s, 2 x 2 H, CH₂O), 4.02 (m, 2 H, O-CH₂), 3.93 (s, 3 H, O-Me), 1.85 (m, 1 H, H-3'), 1.65 (br, 2 H, OH), 1.60 (m, 3 H, H-2', H-7'), 1.28 (m, 6 H, H-4', H-5', H-6'), 0.96 (d, 3 H, Me, J = 7.5 Hz), 0.86 (d, 6 H, Me, J = 7.5 Hz).

### Beispiel D8: Synthese von 2,5-Bishydroxymethyl-4-methoxy-3'-(3,7-dimethyloctyloxy)biphenyl

Synthese analog Beispiel D4. Ausrühren mit heißem Hexan. Man erhielt das Produkt (99% Ausbeute) als farblosen, wachsartigen Feststoff.
Schmelzpunkt: 55°C
¹H NMR (CDCl₃): δ [ppm] = 7.29 (t; 1 H; J = 8 Hz; H-5'), 7.21, 7.12 (2 x s, 2 x 1 H, H-3, H-6), 6.87 (m; 3 H; H-2', H-4', H-6'), 4.70, 4.64 (2 x d, 2 x 2 H, CH₂O, J = 8 Hz), 4.01 (m, 2 H, O-CH₂), 3.93 (s, 3 H, O-Me), 2.29, 1.63 (2 x t, 2 x 1 H, OH, J = 8 Hz), 1.84 (m, 1 H, H-3'), 1.60 (m, 3 H, H-2', H-7'), 1.25 (m, 6 H, H-4', H-5', H-6'), 0.94 (d, 3 H, Me, J = 7.5 Hz), 0.87 (d, 6 H, Me, J = 7.5 Hz).

### E. Halogenierungen gemäß Reaktion C(b)

### Beispiel E1: Synthese von 2,5-Bisbrommethyl-4'-hexyloxybiphenyl:

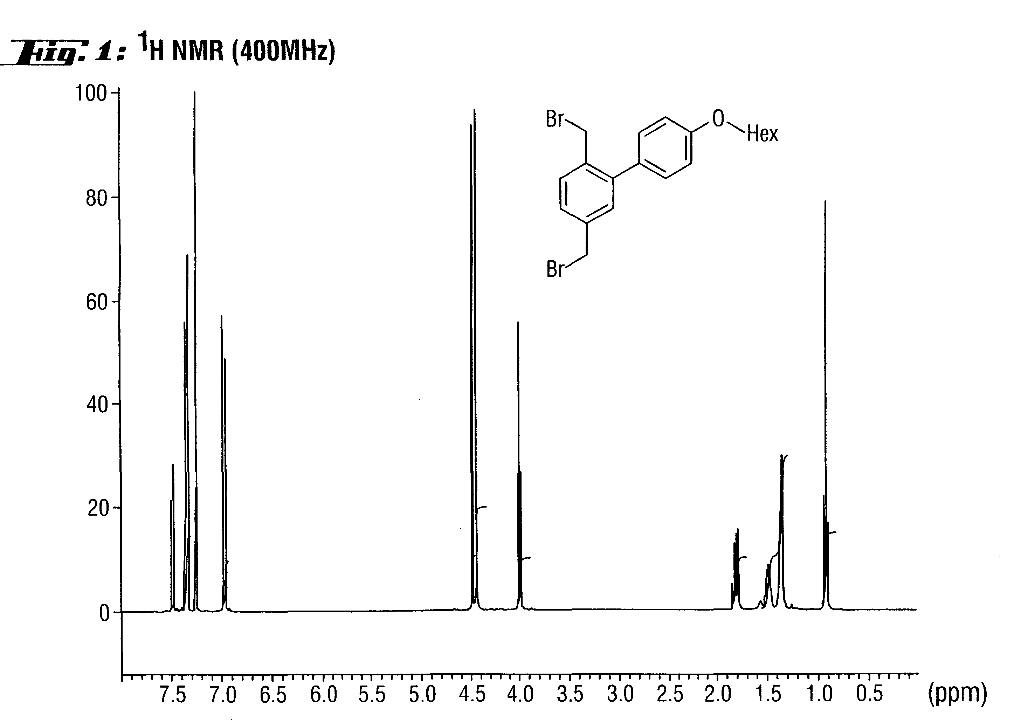

Unter Wasserkühlung wurden in HBr (33%ig in HAc, 36 ml, 200 mmol) 2,5-Bishydroxymethyl-4'-hexyloxybiphenyl (12.6 g, 40 mmol) (vgl. D1) eingerührt. Die zweiphasige, hellbraune und leicht viskose Suspension wurde bei RT unter Schutzgas über Nacht gerührt. Die resultierende Reaktionsmischung schüttelte man mit Chloroform so oft aus, bis die Wasserphase farblos war. Aus der eingeengten organischen Phase erhielt man ein klares, honigfarbenes Öl, welches in der Kälte innerhalb 1-2 Tagen zu einem wachsartigen, trüben Feststoff erstarrte: 16.9 g (96 %); Schmelzpunkt: 38,5° - 40,5° C; Reinheit > 98%.
¹H NMR (400 MHz; CDCl₃): δ [ppm] = 7.49 (d; 1 H; J = 8 Hz; H-6), 7.35 (dd; 1 H; J₁ = 8, J₂ = 2 Hz; H-5), 7.26 (d; 1 H; J = 2 Hz; H-3), 7.36, 6.98 (AA'BB'; 4 H; H-phenyl), 4.48, 4.44 (jeweils: s; 2 H; CH₂-Br), 4.01 (t; 2 H; J = 6.5 Hz; O-CH₂), 1.81 (quint; 2 H; J = 6.9 Hz; O-CH₂-CH₂), 1.50-1.30 (m; 6 H; H-alkyl), 0.92 (t; 3 H; J = 7.0 Hz; CH₃). Das in Abbildung 1 gezeigte 1H-NMR-Spektrum belegt die Reinheit der Verbindung.

### Beispiel E2: Synthese von 2,5-Bischlormethyl-4'-hexyloxybiphenyl:

2,5-Bis(hydroxymethyl)-4'-hexyloxybiphenyl (9.43 g, 30 mmol) (vgl. D1) wurde mit 50 ml Toluol, vermischt mit einem Tropfen Pyridin vorgelegt (ungelöst) und SOCl₂ innerhalb ca. 10 min zugetropft. Nach wenigen Tropfen Zugabe klarte die Suspension auf, was mit einem leichten Temperaturanstieg einherging. Die Lösung wurde anschließend bei 60° Innentemperatur gerührt. Nach 90 Minuten wurde der Ansatz aufgearbeitet. Die Reaktionsmischung wurde nach Abkühlen mit ca. 20 ml Wasser versetzt und dann mit H₂O ausgeschüttelt. Die Wasserphase schüttelte man mit Toluol, vereinigte die organischen Phasen und engte ein: 10,5 g (100%) honigfarbenes, öliges Produkt. Reinheit ca. 90% (¹H-NMR).
¹H NMR (400 MHz; CDCl₃): δ [ppm] = 7.53 (d; 1 H; J = 8 Hz; H-6), 7.38 (dd; 1 H; J₁ = 8, J₂ = 2 Hz; H-5), 7.28 (d; 1 H; J = 2 Hz; H-3), 7.33, 6.97 (AA'BB'; 4 H; H-phenyl), 4.60, 4.53 (jeweils: s; 2 H; CH₂-Cl), 4.01 (t; 2 H; J = 6.9 Hz; O-CH₂), 1.83 (pseudo-quint; 2 H; J = 6.9 Hz; O-CH₂-CH₂), 1.55-1.33 (m; 6 H; H-alkyl), 0.94 (m; 3 H; CH₃).

### Beispiel E3: Synthese von 2,5-Bisbrommethyl-2',5'-dimethylbiphenyl:

In mittels Wasserbad gekühlte HBr (33%ig in HAc, 36 ml, 200 mmol) wurde 2,5-Bishydroxymethyl-2',5'-dimethylbiphenyl (10 g, 41 mmol) (vgl. D3) eingerührt. Die klare Lösung wurde bei RT unter Schutzgas über Nacht eingerührt. Man schüttelte mit Chloroform mehrmals aus, bis die Wasserphase farblos war. Aus der eingeengten organischen Phase erhielt man ein honigfarbenes Öl, welches auch in der Kälte (-18°C) nicht kristallisierte: 14.3 g (94 %); Reinheit > 98%.
¹H NMR (400 MHz; CDCl₃): δ [ppm] = 7.52 (d; 1 H; J = 7.8 Hz; H-6), 7.37 (dd; 1 H; J₁ = 7.8, J₂ = 1.9 Hz; H-5), 7.18 (d; 1 H; J = 7.8 Hz; H-3'), 7.17 (d; 1 H; J = 1.9 Hz; H-3), 7.11 (dd; 1 H; J₁ = 7.7, J₂ = 1.6 Hz; H-4'), 7.00 (d; 1 H; J = 1.7 Hz; H-6'), 4.48, 4.28 (jeweils: AB; 2 H; J_{AB} = 12 Hz; CH₂-Br), 2.35, 2.03 (jeweils: s; 3 H; aryl-CH₃).

### Beispiel E4: Synthese von 2,5-Bischlormethyl-2',5'-dimethylbiphenyl:

Zu 2,5-Bishydroxymethyl-2',5'-dimethylbiphenyl (34.2 g, 141 mmol) wurde SOCl₂ (36.9 g; 22.7 ml, 310 mmol) innerhalb etwa 20 Minuten unter Schutzgas bei Raumtemperatur unter Rühren eingetropft. Am Ende der Zugabe lag eine ölige, leicht trübe Lösung vor. Der Reaktionsansatz wurde 20 Stunden bei Raumtemperatur gerührt, anschließend vorsichtig in 200 ml wäßriger NaHCO₃ - Lösung eingerührt und mit Ethylacetat kräftig verrührt. Nach Phasentrennung schüttelte man die organische Phase mit Wasser aus, bis sie neutral war und zog schließlich nach Trocknen über Na₂SO₄ das Lösungsmittel ab. Reinigung erfolgt durch fraktionierte Vakuumdestillation über etwas NaHCO₃. Man erhielt 27.9 g (65%) Produkt als klares viskoses Öl; Reinheit >99% (Siedepunkt: 135°C bei 0,3 mbar).
¹H NMR (400 MHz; CDCl₃): δ [ppm] = 7.56 (d; 1 H; J = 7.9 Hz; H-6), 7.40 (dd; 1 H; J₁ = 7.9, J₂ = 1.8 Hz; H-5), 7.18 (d; 1 H; J = 1.8 Hz; H-3), 7.16 (d; 1 H; J = 8 Hz; H-3'), 7.11 (dd; 1 H; J₁ = 7.9, J₂ = 1.6 Hz; H-4'), 6.97 (d; 1 H; J = 1.5 Hz; H-6'), 4.60, 4.35 (jeweils: AB; 2 H; J_{AB} = 12 Hz; CH₂-Cl), 2.33, 2.02 (jeweils: s; 3 H; aryl-CH₃).

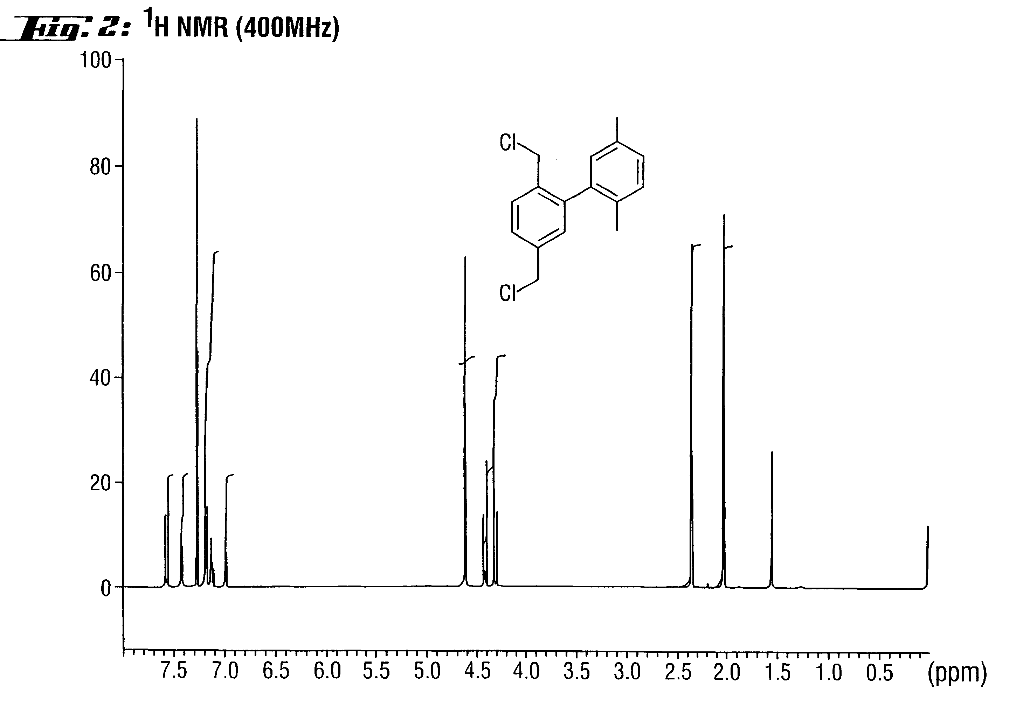

Die Reinheit der erhaltenen Verbindung wird durch das in Abbildung 2 gezeigte ¹H-NMR-Spektrum belegt.

### Beispiel E5: Synthese von 2,5-Bischlormethyl-3'-(3,7-dimethyloctyloxy)biphenyl:

Unter N₂ wurde 2,5-Bishydroxymethyl-3'-(3,7-dimethyloctyloxy)biphenyl (50.7 g, 137 mmol) vorlegt und Thionylchlorid (20 ml, 274 mmol) vorsichtig zugeben. Es wurde noch zweimal (nach 2 und nach 8 Stunden) jeweils 2 ml Thionylchlorid nachgegeben und der Ansatz schließlich insgesamt 20 Stunden bei Raumtemperatur gerührt. Der Ansatz wurde vorsichtig auf wäßrige NaHCO₃-Lösung gegossen, und mit Ethylacetat extrahiert und schließlich wurde die organische Phase noch neutral gewaschen. Nach dem Trocknen über MgSO₄ wurde das Ethylacetat abgezogen und der Ansatz im Vakuum fraktioniert destilliert. Man erhielt das Produkt (39 g, 70 %) als hochviskoses farbloses Öl (Siedepunkt: 212°C bei 0.67 mbar).
¹H NMR (300 MHz; CDCl₃): δ [ppm] = 7.54 (d; 1 H; J = 8.3 Hz; H-6), 7.41 (dd; 1 H; J₁ = 8.2, J₂ = 2.1 Hz; H-5), 7.34 (d; 1 H; J₁ = 8, J₂ = 1 Hz; H-5'), 7.31 (d; 1 H; J = 2 Hz; H-3), 6.94 (m; 3 H; H-2'; H-4', H-6'); 4.61, 4.52 (jeweils: s; 2 H; CH₂Cl), 4.04 (m; 2H; O-CH₂), 1.84 (m; 1 H; O-CH₂-CH₂-CH), 1.72-1.46 (m; 3 H; H-alkyl), 1.38-1.10 (m; 6 H; H-alkyl), 0.94 (d; 3 H; J = 6.7 Hz; CH₃), 0.86 (d; 6 H; J = 6.9 Hz; CH₃).

### Beispiel E6: Synthese von 2,5-Bischlormethyl-4'-(3,7-dimethyloctyloxy)biphenyl

Durchführung analog Beispiel E5; das Produkt (67% Ausbeute) wurde durch Destillation an einer Kurzwegdestille (0.3 mbar, 243°C) als farbloses, hochviskoses Öl erhalten (Reinheit: 99 %).
¹H NMR (CDCl₃): δ [ppm] = 7.52 (d, 1 H, H-6, J = 7.9 Hz), 7.38 (dd, 1 H, H-5, J₁ = 7.9, J₂ = 2 Hz), 7.32, 6.97 (AA'BB', 4 H, H-Aryl), 7.29 (d, 1 H, H-3, J = 2 Hz), 4.59, 4.52 (2 x s, 2 x 2 H, CH₂Cl), 4.04 (m, 2 H, O-CH₂), 1.85 (m, 1 H, H-3'), 1.60 (m, 3 H, H-2', H-7'), 1.30 (m, 6 H, H-4', H-5', H-6'), 0.97 (d, 3 H, Me, J = 7.5 Hz), 0.87 (d, 6 H, Me, J = 7.5 Hz).

### Beispiel E7: Synthese von 2,5-Bischlormethyl-3',4'-bis(2-methylpropyloxy)biphenyl

Durchführung analog Beispiel E5; das Produkt (42% Ausbeute) wurde durch Destillation an einer Kurzwegdestille (0.5 mbar, 240°C) als farbloses, hochviskoses Öl erhalten (Reinheit: 99 %).
¹H NMR (CDCl₃): δ [ppm] = 7.53 (d, 1 H, H-6, J = 7.8 Hz), 7.38 (dd, 1 H, H-5, J₁ = 7.8, J₂ = 2 Hz), 7.31 (d, 1 H, H-3, J = 2 Hz), 6.98 (d, 1 H, H-2', J = 2 Hz), 6.93 (d, 1 H, H-5', J = 8 Hz), ), 6.90 (dd, 1 H, H-6', J₁ = 8, J₂ = 2 Hz), 4.60, 4.53 (2 x s, 2 x 2 H, CH₂Cl), 3.80 (m, 4 H, O-CH₂), 2.16 (m, 2 H, CH), 1.07, 1.04 (2 x t, 2 x 6 H, CH₃, J = 7 Hz).

### Beispiel E8: Synthese von 2,5-Bischlormethyl-4"-(3,7-dimethyloctyloxy)terphenyl

Durchführung analog Beispiel E5; das Produkt (25% Ausbeute) wurde durch Destillation an einer Kurzwegdestille (0.1 mbar, 265°C) als farbloses, hochviskoses Öl erhalten (Reinheit: > 99 %).
¹H NMR (CDCl₃): δ [ppm] = 7.65, 7.45 (AA'BB', 4 H, H-Aryl), 7.58, 7.00 (AA'BB', 4 H, H-Aryl), 7.56 (d, 1 H, H-6, J = 8 Hz), 7.43 (dd, 1 H, H-5, J₁ = 8, J₂ = 2 Hz), 7.35 (d, 1 H, H-3, J = 2 Hz), 4.62, 4.57 (2 x s, 2 x 2 H, CH₂Cl), 4.06 (m, 2 H, O-CH₂), 1.87 (m, 1 H, H-3'), 1.60 (m, 3 H, H-2', H-7'), 1.27 (m, 6 H, H-4', H-5', H-6'), 0.97 (d, 3 H, Me, J = 7.5 Hz), 0.87 (d, 6 H, Me, J = 7.5 Hz).

### Beispiel E9: Synthese von 2,5-Bischlormethyl-4-methoxy-4'-(3,7-dimethyloctyloxy)biphenyl

Durchführung analog Beispiel E5; das Produkt (40% Ausbeute) wurde durch Destillation an einer Kurzwegdestille (0.3 mbar, 265°C) als farbloses, hochviskoses Öl erhalten (Reinheit: 99 %).
¹H NMR (CDCl₃): δ [ppm] = 7.29, 6.95 (AA'BB', 4 H, H-Aryl), 7.27, 7.03 (2 x s, 2 x 1 H, H-3, H-6), 4.65, 4.53 (2 x s, 2 x 2 H, CH₂Cl), 4.04 (m, 2 H, O-CH₂), 3.94 (s, 3 H, O-Me), 1.85 (m, 1 H, H-3'), 1.63 (m, 3 H, H-2', H-7'), 1.28 (m, 6 H, H-4', H-5', H-6'), 0.97 (d, 3 H, Me, J = 7.5 Hz), 0.88 (d, 6 H, Me, J = 7.5 Hz).

### Beispiel E10: Synthese von 2,5-Bischlormethyl-4-methoxy-3'-(3,7-dimethyloctyloxy)biphenyl

Durchführung analog Beispiel E5; das Produkt (25% Ausbeute) wurde durch Destillation an einer Kurzwegdestille (0.2 mbar, 247°C) als farbloses, hochviskoses Öl erhalten. Mehr Produkt konnte aus dem Destillationsrückstand durch Säulenchromatographie gewonnen werden (Reinheit: 99 %).
¹H NMR (CDCl₃): δ [ppm] = 7.32 (t; 1 H; J = 8 Hz; H-5'), 7.30, 7.04 (2 x s, 2 x 1 H, H-3, H-6), 6.93 (m; 3 H; H-2', H-4', H-6'), 4.66, 4.53 (2 x s, 2 x 2 H, CH₂Cl), 4.04 (m, 2 H, O-CH₂), 3.95 (s, 3 H, O-Me), 1.84 (m, 1 H, H-3'), 1.60 (m, 3 H, H-2', H-7'), 1.25 (m, 6 H, H-4', H-5', H-6'), 0.94 (d, 3 H, Me, J = 7.5 Hz), 0.86 (d, 6 H, Me, J = 7.5 Hz).

### F) Oxidationen gemäß Reaktion C(a)

### Beispiel F1: Synthese von 2-(4'-Hexyloxyphenyl)terephthaldehyd:

70 ml Dichlormethan wurden vorgelegt und mit Oxalylchlorid (8.4 g, 5.7 ml, 66 mmol) versetzt und auf -60°C abgekühlt. Dazu wurde innerhalb von 10 Minuten eine Lösung von DMSO (10.2 g, 9.3 ml, 131 mmol) in 30 ml Dichlormethan getropft. Die Mischung wurde 5 Minuten nachgerührt. Dann wurde innerhalb von 15 Minuten eine Lösung aus 2,5-Bis(hydroxymethyl)-4'-hexyloxybiphenyl (10 g, 32 mmol) (vgl. D1) in 70 ml Dichlormethan zugetropft (die Reaktionslösung wurde trüb). Es wurde 10 Minuten nachgerührt, anschließend wurde Triethylamin (15.9 g, 21.8 ml, 157 mmol) zugetropft. Dabei färbte sich die Reaktionslösung gelb, und es bildete sich Niederschlag. Das Aceton/Trockeneis-Bad wurde entfernt und der Ansatz für 2 Stunden bei RT gerührt. Anschließend schwamm heller Feststoff auf der gelben, flüssigen Phase. Die Mischung wurde mit 150 ml Wasser versetzt, 10 Minuten nachgerührt (Feststoff ging in Lösung), die organische Phase wurde abgetrennt, die wäßrige Phase zweimal mit Dichlormethan extrahiert, die vereinigten organischen Phasen anschließend dreimal mit Wasser gewaschen, über Na₂SO₄ getrocknet, abfiltriert und anschließend am Rotationsverdampfer zur Trockene eingeengt. Das gelbe Öl kristallisierte nach einiger Zeit bei RT, es wurde anschließend aus Hexan umkristallisiert. Es dauerte relativ lange, bis das Produkt fest wurde: hellbeiges, mikrokristallines Pulver, 5.67 g (57%), Reinheit ca. 98%ig. Schmelzpunkt: 44.5-45.5°C.
¹H NMR (400 MHz; CDCl₃): δ [ppm] = 10.14 (s; 1 H; 1-CHO), 10.05 (d; 1 H; J = 0.8 Hz; 4-CHO), 8.13 (d; 1 H; J = 7.5 Hz; H-6), 7.96 (d; 1 H; J = 1.5 Hz; H-3), 7.94 (ddd; 1 H; J₁ = 7.7, J₂ = 1.5, J₃ = 0.8 Hz; H-5), 7.33, 7.03 (AA'BB'; 4 H; H-phenyl), 4.03 (t; 2 H; J = 6.7 Hz; O-CH₂), 1.83 (quint; 2 H; J = 6.6 Hz; O-CH₂-CH₂), 1.55-1.35 (m; 6 H; H-alkyl), 0.92 (t; 3 H; J = 7.2 Hz; CH₃).

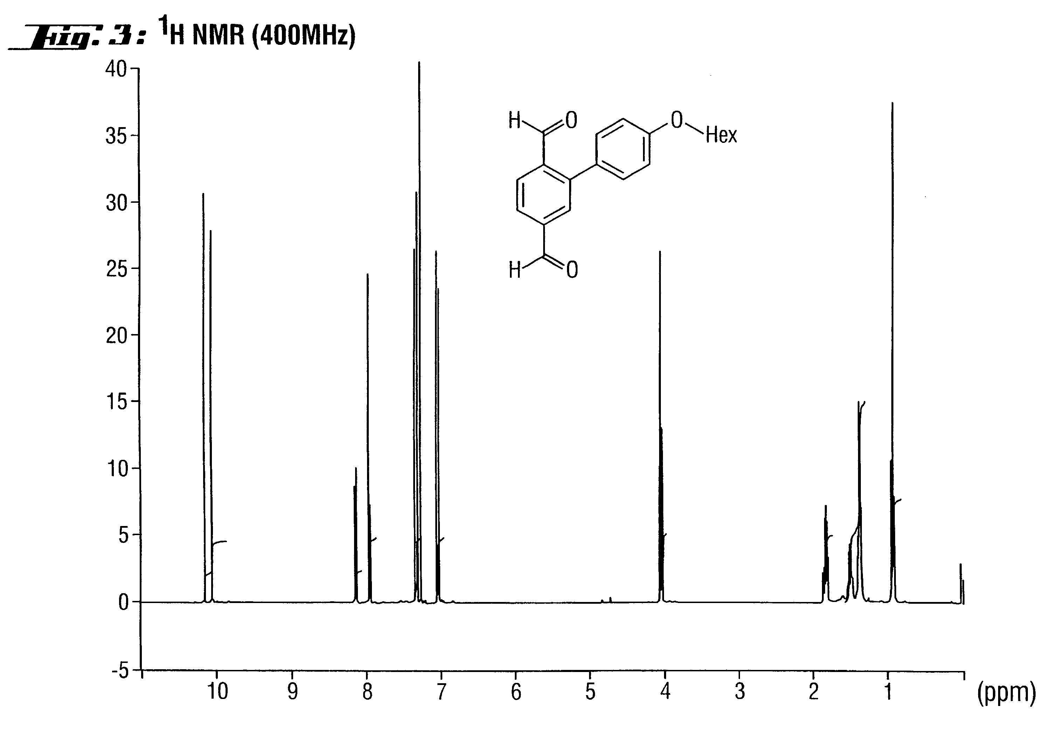

Die Reinheit der Verbindung wird durch das in Abbildung 3 gezeigte ¹H-NMR-Spektrum belegt.

### G. Reaktionen gemäß Reaktion D

### Beispiel G1: Synthese von 2,5-Bis(methylendiethylphosphonat)-4'-hexyloxybiphenyl:

2,5-Bis(chlormethyl)-4'-hexyloxybiphenyl (9.2 g, 26.2 mmol) (vgl. E2) und Triethylphosphit (10.9 g, 11.2 ml, 65.5 mmol) wurden unter Schutzgas gemischt und auf 60°C Ölbadtemperatur erwärmt (ohne Kühler), dabei entwich Chlorethan. Nach 40 Minuten Reaktionszeit wurde langsam mit Kühler erwärmt und anschließend für 3 Stunden bei 190°C gerührt. Anschließend wurde zunächst bei RT dann bei Erwärmung bis 190°C bei ca. 1 mbar getrocknet. Das Rohprodukt wurde in Ethylacetat aufgenommen, mit Wasser extrahiert und schließlich am Rotationsverdampfer erneut vom Lösungsmittel befreit: 13.11 g (90%) leicht bräunliches Öl. Reinheit: ca. 90%ig (¹H-NMR).
¹H NMR (400 MHz; CDCl₃): δ [ppm] = 7.50 (dd; 1 H; J₁ = 8.2, J₂ = 2.5 Hz; H-6), 7.28, 6.93 (AA'BB'; 4 H; H-phenyl), 7.24 (td; 1 H; J₁ = 8.2, J₂ = 2.2 Hz; H-5), 7.16 (m; 1 H; H-3), 3.97 (m; 10 H; P-O-CH₂, Aryl-O-CH₂), 3.17, 3.13 (jeweils: d; 2 H; J = 8 Hz; CH₂-P), 1.82 (m; 2 H; O-CH₂-CH₂), 1.54-1.33 (m; 6 H; H-alkyl), 1.25, 1.22 (jeweils: t; 6 H; J = 6.7 Hz; P-O-CH₂-CH₃), 0.92 (m; 3 H; CH₃).

### V) Vergleichsbeispiele:

### Beispiel V1: Synthese von 2,5-Dimethyl-4'-hexyloxybiphenyl:

Brom-p-xylol (8.3 g, 45 mmol) (vgl. A1 a)), K₂CO₃ (12.4 g, 90 mmol), 70 ml Toluol und 70 ml H₂O wurden vorgelegt und 30 min mit Argon gespült. Anschließend wurde 4-Hexyloxyphenylboronsäure (10 g, 45 mmol), sowie Pd(PPh₃)₄ (0.65 g, 0,56 mmol) unter Schutzgas zugegeben. Das gelb-grünliche, trübe Gemisch wurde unter Schutzgasüberlagerung bei 85° C Innentemperatur für ca. 20 Stunden kräftig gerührt. Nach Phasentrennung wurde die organische Phase mit verdünnter HCl / H₂O ausgeschüttelt (neutral). Die wäßrige Phase schüttelte man mit Toluol aus und vereinigte die organischen Phasen. Nach Abfiltrieren von evtl. Palladium-Resten wurde eingeengt. Zur Reinigung wurde im Vakuum destilliert: Das Produkt erhielt man als hellgelbes Öl (Siedepunkt 117°- 125°C/0,08 mbar) 10.3 g (81%). Reinheit > 95%, (¹H-NMR).
¹H NMR (400 MHz; CDCl₃): δ [ppm] = 7.22, 6.92 (AA'BB'; 4 H; H-phenyl), 7.13 (d; 1 H; J = 8.2 Hz; H-6), 7.04 (m; 2 H; H-3, H-5), 3.99 (t; 2 H; J = 7.2 Hz; O-CH₂), 2.33, 2.23 (jeweils: s; 3 H; Aryl-Me), 1.80 (quint; 2 H; J = 7.0 Hz; O-CH₂-CH₂), 1.50-1.34 (m; 6 H; H-alkyl), 0.92 (m; 3 H; CH₃).

### Beispiel V2: Versuchte Synthese von 2,5-Bisbrommethyl-4'-hexyloxybiphenyl: (in Analogie zu: J. Andersch et al., J. Chem. Soc. Chem. Commun. 1995, 107)

2,5-Dimethyl-4'-hexyloxybiphenyl (9.05 g, 32 mmol), N-Bromsuccinimid (NBS) (11,81 g, 66 mmol) und Azoisobutyronitril (0,5 g, 3,05 mmol) wurden zusammen in CCl₄ (75 ml) vorgelegt und unter Wasserausschluß für 5 Tage am Rückfluß gekocht. Nach zwei Tagen wurde (nach DC-Überprüfung) nochmals ein Äquivalent NBS zugegeben. Der Feststoff wurde abgesaugt, der Niederschlag wurde nochmals mit Tetrachlorkohlenstoff ausgerührt und abgesaugt, laut DC war im Feststoff kein Produkt, die bräunliche Mutterlauge wurde eingeengt. Man erhielt 17.53 g (125 %) eines öligen Rohprodukts. Dieses bestand laut ¹H-NMR aus unterschiedlich halogenierten Verbindungen (es waren sowohl Aryl-CH₃- als auch Aryl-CHBr₂-Gruppen als Nebenprodukte zu erkennen; eine Bromierung der Alkoxykette war ebenfalls nicht auszuschließen.). Es konnte kein Hauptprodukt isoliert werden.

## Patentansprüche

1. Verfahren zur Herstellung eines polymerisierbaren Biarylderivats der Formel (I) 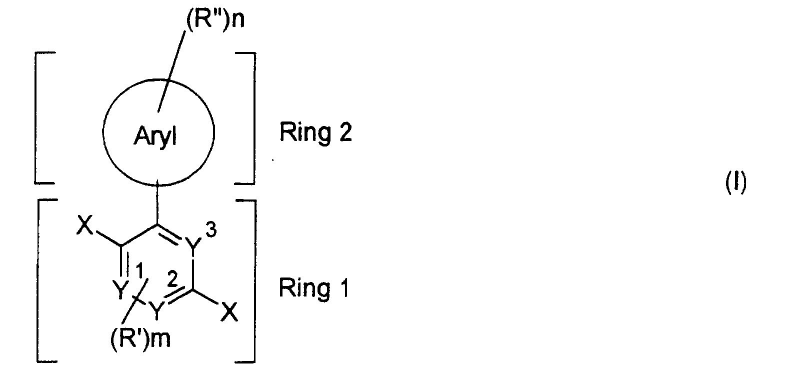 wobei die Symbole und Indizes folgende Bedeutungen haben:
X : -CH₂Z, -CHO;
Y¹, Y², Y³: gleich oder verschieden, CH, N;
Z : gleich oder verschieden, I, Cl, Br, CN, SCN, NCO, PO(OR¹)₂, PO(R²)₂, P(R³)₃⁺A⁻;
Aryl : eine Arylgruppe mit 4 bis 14 C-Atomen;
R', R" : gleich oder verschieden CN, F, Cl, eine geradkettige oder verzweigte oder cyclische Alkyl- oder Alkoxygruppe mit 1 bis 20 C-Atomen, wobei ein oder mehrere nicht benachbarte CH₂-Gruppen durch -O-, -S-, -CO-, -COO-, -O-CO-, -NR⁴-, -(NR⁵R⁶)⁺-A⁻, oder -CONR⁷- ersetzt sein können und wobei ein oder mehrere H-Atome durch F ersetzt sein können, oder eine Arylgruppe mit 4 bis 14 C-Atome, die durch einen oder mehrere, nicht aromatische Reste R' substituiert sein kann;
R¹,R²,R³,R⁴,R⁵,R⁶,R⁷ gleich oder verschieden aliphatische oder aromatische Kohlenwasserstoffreste mit 1 bis 20 C-Atomen, R⁴ bis R⁷ können auch H sein;
A^{⊖} : ein einfach geladenes Anion oder dessen Äquivalent;
m : 0, 1 oder 2;
n : 1, 2, 3, 4 oder 5;
**dadurch gekennzeichnet**,
A. daß man zwei Arylderivate der Formeln (II) und (III), 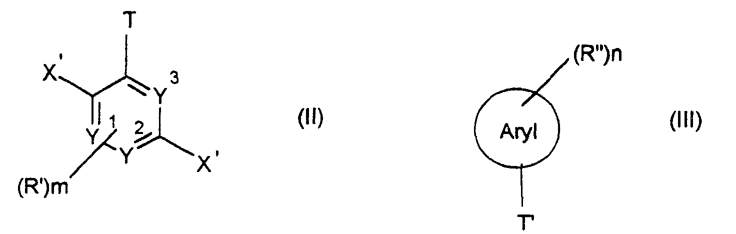 in einem inerten Lösungsmittel in Gegenwart eines Palladiumkatalysators bei einer Temperatur im Bereich von 0°C bis 200°C zu einem Zwischenprodukt der Formel (IV) 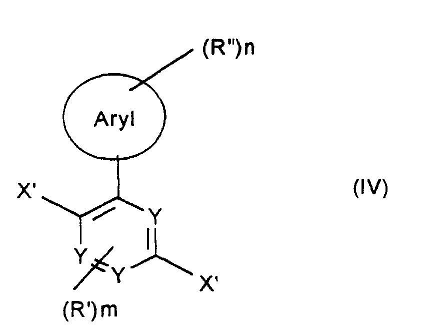 umsetzt,
wobei die Symbole und Indizes die in Formel (I) angegebenen Bedeutungen haben und
X' : CH₂OH oder COOR⁸;
eine der Gruppen T, T': Cl, Br, I oder ein Perfluoralkylsulfonatrest, mit vorzugsweise 1 bis 12 C-Atomen,
und die andere Gruppe T, T': SnR₃, BQ₁Q₂ bedeutet, wobei
Q₁,Q₂ gleich oder verschieden -OH, C₁-C₄-Alkoxy, C₁-C₄-Alkyl, Phenyl, das gegebenenfalls durch C₁-C₄-Alkyl, C₁-C₄-Alkoxy oder Halogen substituiert sein kann, oder Halogen bedeuten oder Q₁ und Q₂ zusammen bilden eine C₁-C₄-Alkylendioxy-Gruppe, die gegebenenfalls durch eine oder zwei C₁-C₄-Alkylgruppen substituiert sein kann, und
R⁸ ist gleich oder verschieden H oder eine geradkettige oder verzweigte Alkylgruppe mit 1 bis 12 Kohlenstoffatomen;
B. falls die Gruppe X' in dem Zwischenprodukt der Formel (IV) COOR⁸ bedeutet (IVa), man mit einem Reduktionsmittel zu einem Zwischenprodukt der Formel (IV) reduziert, bei dem X' CH₂OH bedeutet (IVb), und
C. man das so erhaltene Zwischenprodukt der Formel (IVb) nach einer der folgenden Reaktionen umsetzt:
a) selektive Oxidation zu einer Verbindung der Formel (1) mit X = CHO oder
b) Austausch der OH-Gruppe gegen ein Halogen oder Pseudohalogen mittels nucleophiler Substitution zu einer Verbindung der Formel (I) mit Z = Cl, Br, l, CN, SCN, OCN; und
D. man gegebenenfalls Verbindungen der Formel (I) mit Z = Cl, Br, I, CN, SCN durch Umsetzung mit den entsprechenden phosphororganischen Verbindungen ein Biarylderivat der Formel (I) mit Z = PO(OR¹)₂, PO(R²)₂, P(R³)₃⁺A⁻ überführt.

2. Verfahren nach Anspruch 1, **dadurch gekennzeichnet, daß** T in der Formel (II) I, Br, Cl oder ein Perfluoralkylsulfonatrest mit 1 bis 12 C-Atomen ist, und T' in der Formel (III) BQ₁Q₂ bedeutet, wobei
Q₁,Q₂ gleich oder verschieden -OH, C₁-C₄-Alkoxy, C₁-C₄-Alkyl, Phenyl, das gegebenenfalls durch C₁-C₄-Alkyl, C₁-C₄-Alkoxy oder Halogen substituiert sein kann, oder Halogen bedeuten oder Q₁ und Q₂ zusammen bilden eine C₁-C₄-Alkylendioxy-Gruppe, die gegebenenfalls durch eine oder zwei C₁-C₄-Alkylgruppen substituiert sein kann.

3. Verfahren nach Anspruch 2, **dadurch gekennzeichnet, daß** man die Verbindungen der Formel (II) und (III), eine Base, einen Palladiumkatalysator, der mindestens einen Komplexliganden enthält, in Wasser, ein oder mehrere inerte organische Lösungsmittel oder eine Mischung aus Wasser und einem oder mehreren inerten organischen Lösungsmitteln gibt und bei einer Temperatur im Bereich von 50 bis 150°C umsetzt.

4. Verfahren nach einem oder mehreren der vorhergehenden Ansprüche, **dadurch gekennzeichnet, daß** man eine Verbindung der Formel (II) mit X' = COOR⁸ verwendet.

5. Verfahren nach Anspruch 4, **dadurch gekennzeichnet, daß** man in Schritt B das Zwischenprodukt der Formel (IV) durch
a) Umsetzung mit Li-AlH₄ oder Diisobutylaluminiumhydrid (DIBAL-H), in Tetrahydrofuran (THF) oder Toluol;
b) Umsetzung mit Borhydriden;
c) Umsetzung mit Wasserstoff in Gegenwart eines Katalysators, oder
d) Reaktion mit Natrium oder Natriumhydrid
zu einem Bisalkohol der Formel (IV) umsetzt.

6. Verfahren nach einem oder mehreren der vorhergehenden Ansprüche, **dadurch gekennzeichnet, daß** man in Schritt Ca den Bisalkohol der Formel (IVb) durch
a) Oxidation mit Dimethylsulfoxid/Oxalylchlorid oder
b) Oxidation mit Pyridiniumchlorochromat oder Pyridiniumdichromat
zu einem Bisaldehyd der Formel (I) mit X = CHO umsetzt.

7. Verfahren nach einem oder mehreren der Ansprüche 1 bis 5, **dadurch gekennzeichnet, daß** man in Schritt Cb, den Bisalkohol der Formel (IVb) durch
a) Umsetzung mit HCI oder HBr oder
b) Umsetzung mit Thionylchlorid oder Thionylbromid in eine Verbindung der Formel (Ib) mit X = Cl bzw. Br überführt.

8. Verfahren nach einem oder mehreren der Ansprüche 1 bis 5 und 7, **dadurch gekennzeichnet, daß** man in Schritt D eine Verbindung der Formel (Ib) durch Umsetzung mit einem Trialkylphosphit in ein Bisphosphonat der Formel (Ic) mit X = -PO(R¹)₂ überführt.

9. Polymerisierbares Biarylderivat der Formel (I), 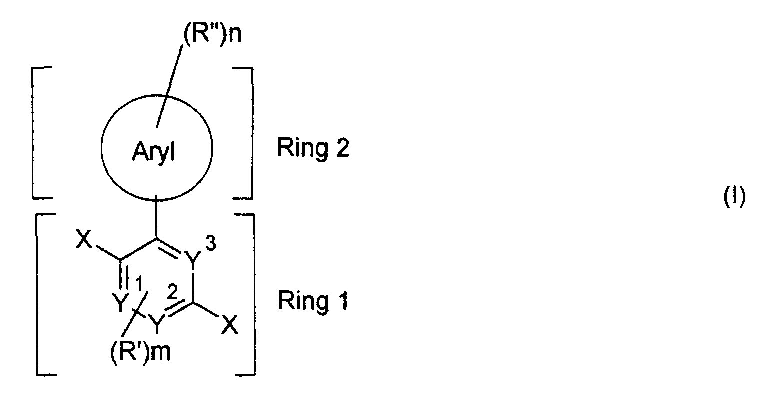 wobei die Symbole und Indizes folgende Bedeutungen haben:
X : -CH₂Z, -CHO;
Y¹, Y², Y³: gleich oder verschieden, CH, N;
Z : gleich oder verschieden, I, Cl, Br, CN, SCN, NCO, PO(OR¹)₂, PO(R²)₂, P(R³)₃⁺A⁻;
Aryl : eine Arylgruppe mit 4 bis 14 C-Atomen;
R', R" : gleich oder verschieden CN, F, Cl, eine geradkettige oder verzweigte oder cyclische Alkyl- oder Alkoxygruppe mit 1 bis 20 C-Atomen, wobei ein oder mehrere nicht benachbarte CH₂-Gruppen durch -O-, -S-, -CO-, -COO-, -O-CO-, -NR⁴-, (NR⁵R⁶)⁺-A⁻, oder -CONR⁷- ersetzt sein können und wobei ein oder mehrere H-Atome durch F ersetzt sein können, oder eine Arylgruppe mit 4 bis 14 C-Atome, die durch einen oder mehrere, nicht aromatische Reste R' substituiert sein kann;
R¹,R²,R³,R⁴,R⁵,R⁶,R⁷ gleich oder verschieden aliphatische oder aromatische Kohlenwasserstoffreste mit 1 bis 20 C-Atomen, R⁴ bis R⁷ können auch H sein;
A^{⊖} : ein einfach geladenes Anion oder dessen Äquivalent;
m : 0, 1 oder 2;
n : 1, 2, 3, 4 oder 5.

10. Polymerisierbares Biarylderivat nach Anspruch 9, wobei die Symbole und Indizes in der Formel (l) folgende Bedeutungen haben:
X -CH₂Z, CHO;
Z Cl, Br, CN, PO(OR¹)₂, PO(R²)₂, P(R³)₃^{⊕}A^{⊖};
Y¹, Y², Y³ CH;
Aryl Phenyl, 1- bzw. 2-Naphthyl, 1-, 2- bzw. 9-Anthracenyl, 2-, 3- bzw. 4-Pyridinyl, 2-, 4- bzw. 5-Pyrimidinyl, 2-Pyrazinyl, 3- bzw. 4-Pyridazinyl, 2-, 3-, 4-, 5-, 6-, 7- bzw. 8-Chinolin, 2- bzw. 3-Thiophenyl, 2- bzw. 3-Pyrrolyl, 2- bzw. 3-Furanyl und 2-(1,3,4-Oxadiazol)yl;
R' gleich oder verschieden geradkettige oder verzweigte Alkoxygruppe mit 1 bis 12 C-Atomen;
R" gleich oder verschieden geradkettige oder verzweigte Alkyl- oder Alkoxygruppe mit 1 bis 12 C-Atomen;
m 0, 1;
n 1,2,3.

11. Verwendung von Biarylderivaten nach Anspruch 9 und/oder 10 zur Herstellung von Polymeren.

## Claims

1. A process for preparing a polymerizable biaryl derivative of the formula (I), 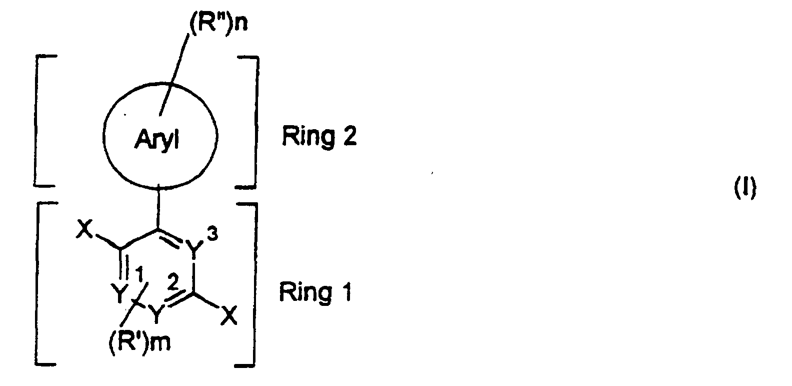 where the symbols and indices have the following meanings:
X: -CH₂Z, -CHO;
Y¹, Y², Y³: identical or different, CH, N;
Z: identical or different, I, Cl, Br, CN, SCN, NCO, PO(OR¹)₂, PO(R²)₂, P(R³)₃⁺A⁻;
Aryl: an aryl group having from 4 to 14 carbon atoms;
R', R": identical or different, CN, F, Cl, a straight-chain or branched or cyclic alkyl or alkoxy group having from 1 to 20 carbon atoms, where one or more nonadjacent CH₂ groups can also be replaced by -O-, -S-, -CO-, -COO-, -O-CO-, -NR⁴-, -(NR⁵R⁶)⁺-A⁻ or -CONR⁷- and one or more H atoms can be replaced by F, or an aryl group having from 4 to 14 carbon atoms which may be substituted by one or more nonaromatic radicals R';
R¹, R², R³, R⁴, R⁵, R⁶, R⁷: identical or different, aliphatic or aromatic hydrocarbon radicals having from 1 to 20 carbon atoms, where R⁴ to R⁷ can also be hydrogen;
A^{⊖}: a singly charged anion or its equivalent;
m: 0, 1 or 2;
n: 1, 2, 3, 4 or 5;
which comprises
A. reacting two aryl derivatives of the formulae (II) and (III), 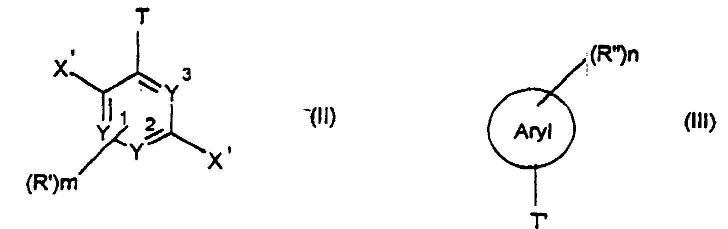 in an inert solvent in the presence of a palladium catalyst at a temperature in the range from 0°C to 200°C to give an intermediate of the formula (IV) 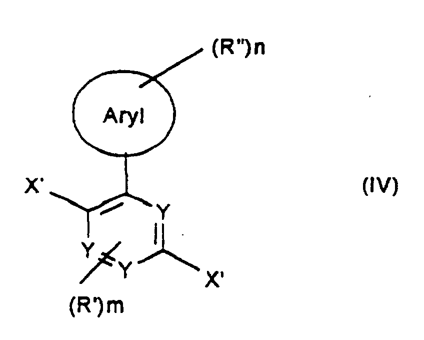 where the symbols and indices have the meanings given in formula (I) and
X': CH₂OH or COOR⁸;
one of the groups T, T': Cl, Br, I or a perfluoroalkylsulfonate radical, preferably having from 1 to 12 carbon atoms,
and the other group T, T': SnR3, BQ1Q2, where
Q₁,Q₂ are identical or different and are each -OH, C₁-C₄-alkoxy, C₁-C₄-alkyl, phenyl which may bear C₁-C₄-alkyl, C₁-C₄-alkoxy or halogen groups as substitutents, or halogen or Q₁ and Q₂ together form a C₁-C₄-alkylenedioxy group which may be substituted by one or two C₁-C₄-alkyl groups; and
R⁸ are identical or different and are each H or a straight-chain or branched alkyl group having from 1 to 12 carbon atoms;
B. if the group X' in the intermediate of the formula (IV) is COOR⁸ (IVa), reducing this by means of a reducing agent to give an intermediate of the formula (lV) in which X' is CH₂OH (IVb), and
C. reacting the resulting intermediate of the formula (IVb) according to one of the following reactions:
a) selective oxidation to form a compound of the formula (I) where X = CHO or
b) replacement of the OH group by a halogen or pseudohalogen by means of nucleophilic substitution to form a compound of the formula (I) where Z = Cl, Br, I, CN, SCN, OCN; and
D. if desired, converting compounds of the formula (I) where Z = Cl, Br, I, CN, SCN into a biaryl derivative of the formula (I) where Z = PO(OR¹)₂, PO(R²)₂, P(R³)₃⁺A⁻ by reaction with the corresponding organophosphorus compounds.

2. The process as claimed in claim 1, wherein T in the formula (II) is I, Br, Cl or a perfluoroalkylsulfonate radical having from 1 to 12 carbon atoms, and T' in the formula (III) is BQ₁Q₂, where
Q₁,Q₂ are identical or different and are each -OH, C₁-C₄-alkoxy, C₁-C₄-alkyl, phenyl which may bear C₁-C₄-alkyl, C₁-C₄-alkoxy or halogen as substituents, or halogen or Q₁ and Q₂ together form a C₁-C₄-alkylenedioxy group which may be substituted by one or two C₁-C₄-alkyl groups.

3. The process as claimed in claim 2, wherein the compounds of the formula (II) and (III), a base and a palladium catalyst; comprising at least one complexing ligand are added to water, one or more inert organic solvents or a mixture of water and one or more inert organic solvents and reacted at a temperature in the range from 50 to 150°C.

4. The process as claimed in one or more of the preceding claims, wherein a compound of the formula (II) in which X' = COOR⁸ is used.

5. The process as claimed in claim 4, wherein, in step B, the intermediate of the formula (IV) is converted into a bisalcohol of the formula (IV) by
a) reaction with Li-AlH4 or diisobutylaluminum hydride (DIBAL-H) in tetrahydrofuran (THF) or toluene;
b) reaction with boron hydrides;
c) reaction with hydrogen in the presence of a catalyst, or
d) reaction with sodium or sodium hydride.

6. The process as claimed in one or more of the preceding claims, wherein, in step Ca, the bisalcohol of the formula (IVb) is converted into a bisaldehyde of the formula (I) with X = CHO by
a) oxidation using dimethyl sulfoxide/oxalyl chloride or
b) oxidation using pyridinium chlorochromate or pyridinium dichromate.

7. The process as claimed in one or more of claims 1 to 5, wherein, in step Cb, the bisalcohol of the formula (IVb) is converted into a compound of the formula (Ib) in which X = Cl or Br by
a) reaction with HCl or HBr or
b) reaction with thionyl chloride or thionyl bromide.

8. The process as claimed in one or more of claims 1 to 5 and 7, wherein, in step D, a compound of the formula (Ib) is converted into a bisphosphonate of the formula (Ic) with X = -PO(R¹)₂ by reaction with a trialkyl phosphite.

9. A polymerizable biaryl derivative of the formula (I), 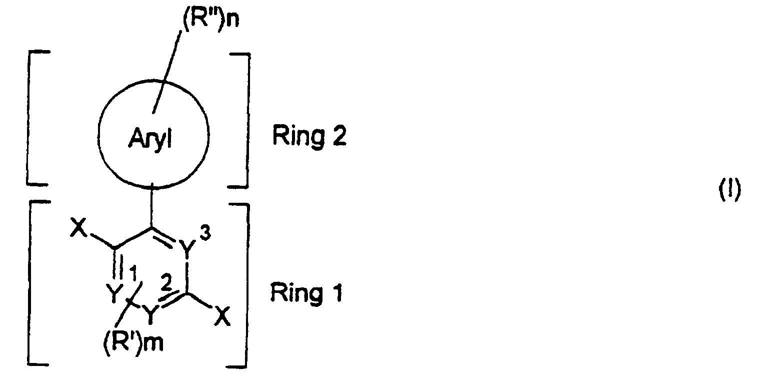 where the symbols and indices have the following meanings:
X: -CH₂Z, -CHO;
Y¹, Y², Y³: identical or different, CH, N;
Z: identical or different, I, Cl, Br, CN, SCN, NCO, PO(OR1)2, PO(R2)2, P(R3)3⁺A⁻;
Aryl: an aryl group having from 4 to 14 carbon atoms;
R', R": identical or different, CN, F, Cl, a straight-chain or branched or cyclic alkyl or alkoxy group having from 1 to 20 carbon atoms, where one or more nonadjacent CH2 groups can also be replaced by -O-, -S-, -CO-, -COO-, -O-CO-, -NR⁴-, (NR⁵R⁶)⁺-A⁻ or -CONR⁷- and one or more H atoms can be replaced by F, or an aryl group having from 4 to 14 carbon atoms which may be substituted by one or more nonaromatic radicals R';
R¹, R², R³, R⁴, R⁵, R⁶, R⁷: identical or different, aliphatic or aromatic hydrocarbon radicals having from 1 to 20 carbon atoms, where R⁴ to R⁷ can also be hydrogen;
A^{⊖}: a singly charged anion or its equivalent;
m: 0,1 or 2;
n: 1, 2, 3, 4 or 5.

10. A polymerizable biaryl derivative as claimed in claim 9, wherein the symbols and indices in the formula (I) have the following meanings:
X: -CH₂Z, CHO;
Z: Cl, Br, CN, PO(OR¹)₂, PO(R²)₂, P(R³)₃^{⊕}A^{⊖};
Y¹, Y², Y³: CH;
Aryl: phenyl, 1- or 2-naphthyl, 1-, 2- or 9-anthracenyl, 2-, 3- or 4-pyridinyl, 2-, 4- or 5-pyrimidinyl, 2-pyrazinyl, 3- or 4-pyridazinyl, 2-, 3-, 4-, 5-, 6-, 7- or 8-quinolyl, 2- or 3-thiophenyl, 2- or 3-pyrrolyl, 2- or 3-furanyl or 2-(1,3,4-oxadiazol)yl;
R': identical or different, straight-chain or branched alkoxy group having from 1 to 12 carbon atoms;
R": identical or different, straight-chain or branched alkyl or alkoxy group having from 1 to 12 carbon atoms;
m: 0, 1;
n: 1, 2, 3.

11. The use of a biaryl derivative as claimed in claim 9 and/or 10 for preparing polymers.

## Revendications

1. Procédé de préparation d'un dérivé biaryle polymérisable de formule (I) 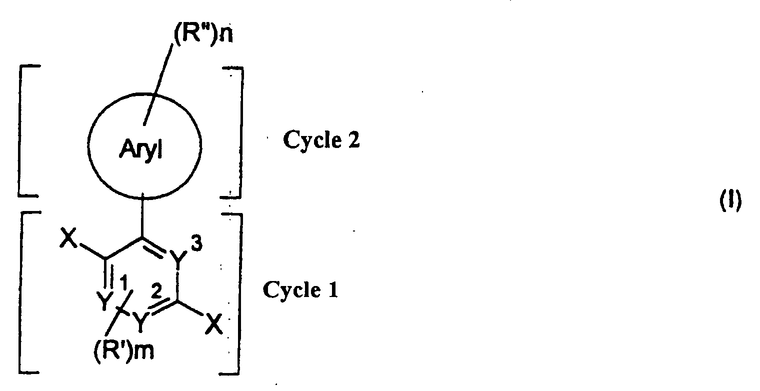 dans laquelle les symboles et indices ont les significations suivantes :
X représente -CH₂Z, -CHO ;
Y¹, Y², Y³, identiques ou différents, représentent CH, N ;
Z identiques ou différents, représentent I, Cl, Br, CN, SCN, NCO, PO(OR¹)₂, PO(R²)₂, P(R³)₃⁺A⁻ ;
Aryle représente un groupe aryle ayant de 4 à 14 atomes de carbone ;
R', R" représentent, identiques ou différents, CN, F, Cl, un groupe alkyle ou alcoxy à chaîne linéaire ou ramifiée ou cyclique ayant de 1 à 20 atomes de carbone, dans lequel un ou plusieurs groupes -CH₂- non voisins peuvent être remplacés par -O-, -S-, -CO-, -COO-, -O-CO-, -NR⁴-, -(NR⁵R⁶)⁺-A⁻ ou -CONR⁷- et dans lequel un ou plusieurs 1 atomes H peuvent être remplacés par F, ou un groupe aryle ayant de 4 à 14 atomes de carbone, qui peut être substitué par un ou plusieurs groupes R' non aromatiques ;
R¹, R², R³, R⁴, R⁵, R⁶, R⁷ identiques ou différents, représentent des groupes hydrocarbonés aliphatiques ou aromatiques avec de 1 à 20 atomes de carbone, R⁴ à R⁷ peuvent aussi être H ;
A⁻ représente un anion de charge unitaire ou son équivalent ;
m représente 0, 1 ou 2 ;
n représente 1, 2, 3, 4 ou 5 ;
**caractérisé en ce que**
A. on met à réagir deux dérivés aryle des formules (II) et (III), 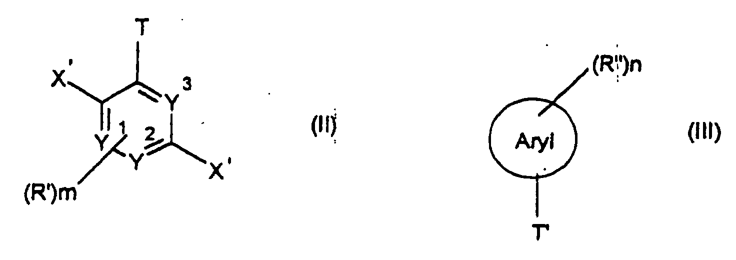 dans un solvant organique inerte el présence d'un catalyseur au palladium à une température dans la gamme de 0°C à 200°C pour obtenir un produit intermédiaire de formule (IV) 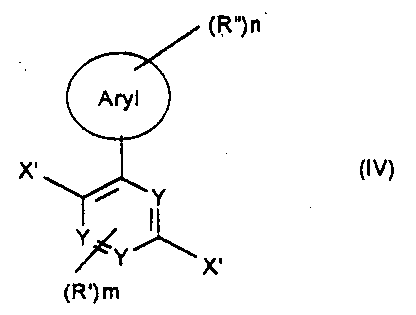 dans lesquelles les symboles et indices ont les significations indiquées à la formule (I) et
X' représente CH₂OH ou COOR⁸ ;
un des groupes T, T' représente Cl, Br, I ou un résidu perfluoroalkylsulfonàte, avec de préférence 1 à 12 atomes de carbone,
et l'autre groupe T, T' représente SnR₃, BQ₁Q₂, dans laquelle
Q₁, Q₂ identiques ou différents, représentent -OH, un groupe alcoxy en C₁-C₄, alkyle en C₁-C₄, phényle, qui peut être éventuellement substitué par un groupe alkyle en C₁-C₄, alcoxy en C₁-C₄ ou halogène, ou représentent un atome d'halogène ou Q₁ et Q₂ forment ensemble un groupe alkylènedioxy en C₁-C₄, qui peut être éventuellement substitué par un ou deux groupes alkyle en C₁-C₄, et
R⁸ représente, identiques ou différents, H ou un groupe alkyle à chaîne linéaire ou ramifiée ayant de 1 à 12 atomes de carbone ;
B. dans le cas où X' dans le produit intermédiaire de formule (IV) représente COOR⁸ (IVa), on réduit avec un agent réducteur en un produit intermédiaire de formule (IV), dans laquelle X' représente CH₂OH (IVb), et
C. on met à réagir le produit intermédiaire ainsi obtenu de formule (IVb) selon une des réactions suivantes :
a) oxydation sélective pour obtenir un composé de formule (I) avec X = CHO ou
b) échange du groupe OH contre un halogène ou un pseudo-halogène au moyen d'une substitution nucléophile pour obtenir un composé de formule (I) avec Z = Cl, Br, I, CN, SCN, OCN ; et
D. on transforme éventuellement les composés de formule (I) avec Z = Cl, Br, I, CN, SCN par réaction avec les composés phosphoroorganiques correspondants en un dérivé biaryle de formule (I) avec Z = PO(OR¹)₂, PO(R²)₂, P(R³)₃⁺A⁻.

2. Procédé selon la revendication 1, **caractérisé en ce que** T dans la formule (II) représente I, Br, Cl ou un résidu perfluoroalkylesulfonate avec de 1 à 12 atomes de carbone, et T' dans la formule (III) représente BQ₁Q₂, dans laquelle
Q₁, Q₂ identiques ou différents, représentent -OH, un groupe alcoxy en C₁-C₄, alkyle en C₁-C₄, phényle, qui peut être éventuellement substitué par un groupe alkyle en C₁-C₄, alcoxy en C₁-C₄ ou halogène, ou représentent un atome d'halogène ou Q₁ et Q₂ forment ensemble un groupe alkylènedioxy en C₁-C₄, qui peut être éventuellement substitué par un ou deux groupes alkyle en C₁-C₄.

3. Procédé selon la revendication 2, **caractérisé en ce qu'**on ajoute les composés de formule (II) et (III), une base, un catalyseur au palladium, qui contient au moins un ligand complexe, dans l'eau, un ou plusieurs solvants organiques inertes ou un mélange d'eau et d'un ou de plusieurs solvants organiques inertes et on met à réagir à une température dans la gamme de 50 à 150°C.

4. Procédé selon une ou plusieurs des revendications précédentes, **caractérisé en ce qu'**on utilise un composé de formule (II) avec X' = COOR⁸.

5. Procédé selon la revendication 4, **caractérisé en ce qu'**on met à réagir à l'étape B le produit intermédiaire de formule (IV) par
a) Réaction avec Li-AlH₄ ou l'hydrure de diisobutyl-aluminium (DIBAL-H), le tétrahydrofurane (THF) ou le toluène ;
b) Réaction avec des borohydrures ;
c) Réaction avec l'hydrogène en présence d'un catalyseur, ou
c) Réaction avec le sodium ou l'hydrure de sodium
pour obtenir un bisalcool de formule (IV).

6. Procédé selon une ou plusieurs des revendications précédentes, **caractérisé en ce qu'**on 'met à réagir à l'étape Ca le bisalcool de formule (IVb) par
a) Oxydation avec le diméthylsulfoxyde/chlorure d'oxalyle ou
b) Oxydation avec le chlorochromate de pyridinium ou le dichromate de pyridinium
pour obtenir un bisaldéhyde de formule (I) avec X = CHO.

7. Procédé selon une ou plusieurs des revendications 1 à 5, **caractérisé en ce qu'**on transforme à l'étape Cb, le bisalcool de formule (IVb) par
a) Réaction avec HCl ou HBr ou
b) Réaction avec le chlorure de thionyle ou le bromure de thionyle pour obtenir un composé de formule (Ib) avec X = Cl ou Br.

8. Procédé selon une ou plusieurs des revendications 1 à 5 et 7, **caractérisé en ce qu'**on transforme à l'étape D un composé de formule (Ib) par réaction avec un triphosphite d'alkyle en un bisphospnonate de formule (Ic) avec X = -PO(R¹)₂.

9. Dérivé biaryle polymérisable de formule (I), 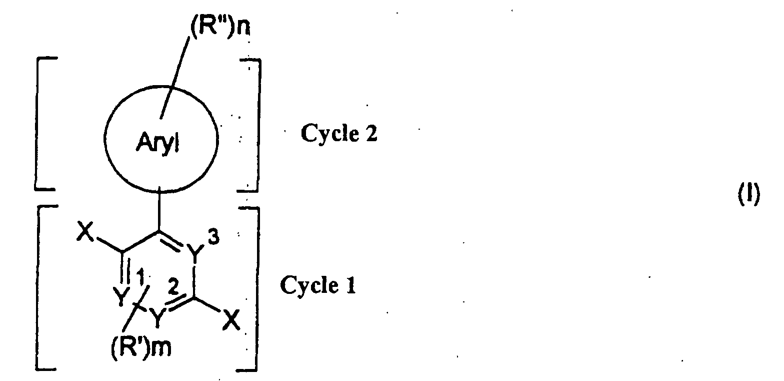 dans laquelle les symboles et indices ont le significations suivantes :
X représente -CH₂Z, -CHO ;
Y¹, Y², Y³, identiques ou différents, représentent CH, N ;
Z identiques ou différents, représentent I, Cl, Br, CN, SCN, NCO, PO(OR¹)₂, PO(R²)₂, P(R³)₃⁺A⁻ ;
Aryle représente un groupe aryle ayant de 4 à 14 atomes de carbone ;
R', R" représentent, identiques ou différents, CN, F, Cl, un groupe alkyle ou alcoxy à chaîne linéaire ou ramifiée ou cyclique ayant de 1 à 20 atomes de carbone, dans lequel un ou plusieurs groupes -CH₂- non voisins peuvent être remplacés par -O-, -S-, -CO-, -COO-, -O-CO-, -NR⁴-, -(NR⁵R⁶)⁺-A⁻ ou -CONR⁷- et dans lequel un ou plusieurs atomes H peuvent être remplacés par F, ou un groupe aryle ayant de 4 à 14 atomes de carbone, qui peut être substitué par un ou plusieurs groupes R' non aromatiques ;
R¹, R², R³, R⁴, R⁵, R⁶, R⁷ identiques ou différents, représentent des groupes hydrocarbonés avec de 1 à 20 atomes de carbone, R⁴ à R⁷ peuvent aussi être H ;
A⁻ représente un anion de charge unitaire ou son équivalent ;
m représente 0, 1 ou 2 ;
n représente 1, 2, 3, 4 ou 5.

10. Dérivé biaryle polymérisable selon la revendication 9, dans lequel les symboles et indices dans la formule (I) ont les significations suivantes :
X représente -CH₂Z, -CHO ;
Z représente Cl, Br, CN, PO(OR¹)₂, PO(R²)₂, P(R³)₃⁺A⁻;
Y¹, Y², Y³ représentent CH ;
Aryle représente un groupe phényle, 1- ou 2-naphtyle, 1-, 2- ou 9-anthracényle, 2-, 3- ou 4-pyridinyle, 2-, 4- ou 5-pyrimidinyle, 2-pyrazinyle, 3- ou 4-pyridazinyle, 2-, 3-, 4-, 5-, 6-, 7- ou 8-quinoléine, 2- ou 3-thiophényle, 2- ou 3-pyrrolyle, 2- ou 3-furanyle et 2-(1,3,4-oxadiazolyle);
R représentent, identiques ou différents, un groupe alcoxy à chaîne linéaire ou ramifiée ayant de 1 à 12 atomes de carbone ;
R" représentent, identiques ou différents, un groupe alkyle ou alcoxy ayant de 1 à 12 atomes de carbone ;
m représente 0, 1 ;
n représente 1, 2, 3.

11. Utilisation de dérivés biaryle selon la revendication 9 et/ou 10 pour la fabrication de polymères.
